# EUROPEAN PATENT APPLICATION

(11) **EP 3 305 335 A1**
(43) Date of publication of application: **11.04.2018**
(21) Application number: 16802494.1
(22) Date of filing: 26.05.2016
(51) Int. Cl.: A61L 15/22, A61L 15/34, A61L 15/18

(54) **MULTIFUNCTIONAL COMPOUND SKIN OR WOUND DRESSING AS REGENERATIVE SKIN SUBSTITUTE**

(30) Priority: 29.05.2015 US 201562168124 P; 29.05.2015 US 201562168184 P
(71) Applicant: Chen, Shirley X., Sunnyvale, California 94087 (US); Xiong, Guangming, Hangzhou, Zhejiang 310000 (CN)
(72) Inventor: Chen, Shirley X., Sunnyvale, California 94087 (US); Xiong, Guangming, Hangzhou, Zhejiang 310000 (CN)
(74) Representative: Loo, Chi Ching
(86) International application number: PCT/CN2016/083460
(87) International publication number: WO 2016/192571

(57) **Abstract**

Provided is a new biomimetic skin material with multiple functions and high efficacy. With its unique composite network structure of micro-cavities and microfilaments, the material can mimic the skin barrier function: being capable of shielding contamination, moisture absorption, moisture retention, breathability, pliability and adhesion. The material is capable of not only absorbing wound exudates to facilitate rapid, non-invasive autolytic debridement and bi-directional regulation to ensure sufficient drainage, but also targetedly and compartmentally retaining exudates in the dressing so as to maintain a semi-occlusive local environment with a physiological moisture balance, which is conducive to intercellular and intracellular signal transduction, promoting migration of autologous fibroblasts and epithelial cells, regeneration of granulation tissue and epithelium, accelerating physiological healing and restoration, and reducing recurrence. It has been clinically proven that the dressing material can accelerate the healing of acute skin injuries and chronic ulcer wounds, achieve an excellent healing quality, and avoid or minimize traumatic skin grafting so as to reduce the pain and scarring of patients, as well as to restore the physiological function and appearance of the body.

## Description

This Application claims priority to U.S. Provisional Application Serial No. 62/168,124, filed May 29, 2015, and to U.S. Provisional Application Serial No. 62/168,184, filed May 29, 2015, which are hereby incorporated by reference in their entirety.

### Technical Area of the Invention

This invention relates to materials for protecting and healing skin and wounds, as well as methods of preparation and use thereof, and especially relates to multifunctional skin and wound dressings as regenerative skin substitutes, and applications in areas such as regenerative medicine, wound protection, wound care, plastic surgery and medical cosmetology.

### Background of the Invention

Wound healing is an extremely well-regulated and complex process. Acute wounds usually follow a well-defined process described as: coagulation; inflammation; cell proliferation and repair of the matrix; epithelialization and remodeling of scar tissue. These reactions are mediated by cytokines, chemokines, and growth factors, as well as the effects of these mediators on cellular receptors. The shape and location of the wound determine how well the wound will heal. Complications result from interference with wound healing. These factors may include poor nutrition, decreased blood supply, tissue trauma, denervation, and infection. Complications that may result include hypertrophic scars and keloids, contracture, dehiscence, excess granulation, and adhesions.

Chronic wounds are the result of an inadequate repair process that is unable to restore anatomic and functional integrity in an appropriate length of time. Chronic wounds are rarely seen in individuals who are otherwise healthy. In fact, chronic wound patients frequently suffer from chronic diseases such as diabetes, obesity, cardiovascular diseases, renal diseases, and cancer. Often disguised as a comorbid condition, chronic wounds represent a silent epidemic that affects a large fraction of the world population and poses major and gathering threat to the public health and economy. One of the serious consequences of long-term non-healing of diabetic foot ulcers, especially for those patients with concomitant neuropathy and peripheral vascular disease, is amputation. It is estimated that annually there are over a million diabetics worldwide who need amputation, which means there would be by average one amputation operation occurring every 30 seconds. The five-year survival rate of a single-limb amputee is only 50%. On the other hand, with an aging population worldwide, chronic wound care related to the elderly's bedsores, lower extremity ulcers have become an increasingly serious health problem. It is claimed that an excess of US$25 billion is spent annually on treatment of chronic wounds (Brem H, et al. MolMed 2007;13:30-9). Other than the developed countries such as the United States, the burden of treating chronic wounds is also growing rapidly due to increasing health care costs, an aging population and, a sharp rise in the incidence of diabetes and obesity worldwide.

The most common chronic wounds are lower extremity ulcers. Chronic venous insufficiency (CVI) accounts for 80% to 90% of lower extremity ulcers and affects 2% to 5% of the population (Kane DP. Chronic wound healing and chronic wound management. In: Chronic Wound Care: A Clinical Source Book for Healthcare Professionals. 4th ed. Malvern, PA: HMP Communications; 2007: 11-23). Other types of non-healing wounds are surgical, diabetic, arterial, bums, dermatitis, vasculitis, and radiation.

At a physiological level, chronic wounds are characterized prolonged inflammation, bacterial bioburden, and ischemia. Within the molecular environment of a chronic wound there exist multiple components that are not conducive to healing, such as high levels of inflammatory cytokines, proteases, and low levels of growth factors. These changes terminate the healing process and increase the potential for septic infections. Addressing the issues that might be responsible for the physiological wound changes may restart healing.

There are a wide variety of wound care products on the market, including those for basic wound care (tapes; dry dressings; cleansing); advanced wound care (films; foam dressings; collagen; alginates; hydrocolloids; hydrogels; super absorbers); bio-active wound care (artificial skin and skin substitutes); and therapy devices (negative pressure wound therapy devices; pressure relief devices; electrical stimulation devices; ultra-violet devices; oxygen and hyperbaric oxygen equipment; whirlpool therapy devices; electromagnetic therapy devices; and ultrasound devices.

Among the variety of treatment modalities, topical treatment of the wound bed is the most important aspect of wound care. Many variety of wound care topical products have been developed to address different components of the complex wound healing process, including, antibacterial/anti-infective wound dressing, moist wound dressing, bio-actives such as growth factor-containing cream, acellular matrix with preserved extracellular bioactive factors, amniotic membrane and living cell-based skin-substitutes.

One of the essential local factors for promoting wound healing is to keep the wound bed moist. It is well recognized that moist wound healing is associated with faster healing, better tissue quality with less scarring, and less pain. However, overhydration can cause maceration. In creating a moist environment, a dressing soothes exposed nerve endings by bathing them in wound secretions, thereby minimizing or eliminating pain and allowing healing to progress more naturally. Dressings that promote a moist wound environment include films, foams, alginates, hydrocolloids, hydrofibers and hydrogels. Hydrocolloids achieve a moist wound environment by gelling with wound fluid above the wound bed. Foams have a greater absorbency capacity than films and hydrocolloids; however, where wound exudation is low or has decreased through treatment, some foams, with the exception of those deemed 'atraumatic', have the disadvantage of sticking to the wound bed. For the uninitiated, dissolved hydrocolloid matrix looks like pus or infection; and they often produce a characteristic odor. Hydrocolloid dressings, pastes and powders were difficult to remove from cavity and undermined wounds. Alginates, when in contact with wound fluid, transform from fibers into a gel which provides non-adherent wound contact and a moist environment for healing. Hydrogels have a high water content in a lattice gel, so not only are they non-adherent but they also provide the wound with adequate moisture. However, hydrogels require a secondary dressing. If used inappropriately or not changed when needed, alginates are at risk of super-saturation, which may macerate the surrounding skin, or they may dry out and adhere to the wound tissue.

For the "bioactive" wound care products, they are generally very expensive (up to $1000 per application). Living cell-based skin substitutes (such as APLIGRAF, DERMAGRAFT, etc.) require a well-prepared wound bed: infection-free and healthy granulation tissue. In the real world of clinical treatment of patients with challenging wounds, the wound is usually complicated with prolonged inflammation, ischemia, eschar/nonviable tissue/fibrous tissue, slough, copious exudate and antibiotic treatment-resistant bacterial biofilms. Sometimes grafting of the skin substitutes (even used in conjunction with other advanced wound care modalities) would fail due to loss of viability of the cells under such the wound bed conditions after spending a large sum of money. In addition, proper handling of many bioactive wound care products and equipment requires extensive technical training of the healthcare provider, sometimes surgeons, most of the times nurses and/or family caregivers.

On the other hand, excessive deposition of collagen as a result of over stimulation of fibroblasts or desiccation of wound bed can result in extensive scarring, which can have profound functional and aesthetic consequences. Hypertrophic scarring commonly occurs following bums. Scars may have long lasting functional, cosmetic as well as psychological consequences for the patient. Both normal and hypertrophic scars remain very difficult to prevent and to treat.

Thus, there exists a great need in the market for high-performance and highly cost-effective innovative products and materials for skin regeneration and wound healing, so as to simplify and streamline the wound treatment process with accelerated healing rate, improved healing quality with reduced scarring, and to prevent amputation, which, in return, would reduce the pain and suffering of patients and the financial burden of health care systems.

### Summary of the Invention

The present invention provides innovative dressing materials that could be used to heal a wide variety of acute and chronic wounds. The dressing is a novel composite material possessing the properties and attributes of an effective skin substitute to protect the wound bed, being soft and pliable to conform to the contours of a wound bed, non-adherent to wound bed upon removal without damaging the nascent tissue and disturbing the kinetics of healing, and to protect the nerve ends of wound bed thus reducing the pain of the patient, and functioning as a semi-occlusive and breathable skin barrier to create an ideal moisture-balanced microenvironment conducive to regenerative wound healing.

The dressing material is also multifunctional-being capable of non-invasively/non-surgically facilitates autolytic debridement of non-viable or necrotic tissue to control microbial infection so as to reduce bioburden while minimizing damage to the healthy live tissue *in situ,* providing an anti-oxidative relief, and stimulating microcirculation and tissue regeneration, including capillary and nerve regeneration. Such dressing materials have been clinically demonstrated to accelerate healing of acute and chronic wounds with superior healing quality and minimal scarring.

In one aspect of the present invention, a composite skin or wound dressing material is provided. In one embodiment, the composite dressing material comprises an active layer of a porous, polymeric substrate impregnated with a semi-solid hydrophobic composition, wherein the composition comprises fatty acid and solid inorganic particles embedded in the matrix of the polymeric substrate.

Further, in an embodiment of the invention, the semi-solid hydrophobic composition is mechanically processed so as to allow the fatty acid to substantially evenly soak through the matrix of the polymeric substrate, and the solid inorganic particles substantially evenly get embedded in the matrix of the polymeric substrate.

According to the embodiment, the semi-solid hydrophobic composition is substantially evenly impregnated in the matrix of the polymeric substrate so as to form a layer of 0.1-10 mm in thickness.

In an embodiment of the invention, the polymeric substrate is a material with a net structure comprising fiber bundles, each of the fiber bundles composed of multiple microfilaments.

Further, there is interstitial space among the multiple microfilaments.

Optionally, the longest cross diameter of the microfilament is 0.01-20 µm.

According to the invention, the porous polymeric substrate is woven or non-woven fiber selected from the group consisting of cotton, silk, linen, polyester, nylon, polyamide, polypropylene, polyurethane, polytetrafluoroethylene, rayon, bamboo fiber, bamboo viscose, corn, soy, alginate, chitin, chitosan, hyaluronan, and animal protein.

In another embodiment of the invention, the semi-solid hydrophobic composition comprises less than 5% of water in weight based on the total weight of the dressing material.

Optionally, the semi-solid hydrophobic composition further comprises one or more of a solidifier, a bioabsorbable composition or hydrogel.

According to the invention, the concentration of the fatty acid is 20-80% by weight based on the total weight of the dressing material.

Further, according to the invention, the solid inorganic particles may be one or more of the inorganic compounds selected from the group consisting of talc, silicon dioxide, aluminum oxide, magnesium oxide, zinc oxide, iron oxides, calcium carbonate, calcium chloride, calcium, calcium sulfate, and titanium dioxide.

In another embodiment of the average size of the particles is preferably 0.01 - 50 µm. Alternatively, at least 80% of the solid inorganic particles has the size of less than 50 or 30 µm.

Optionally, according to the invention, the concentration of the solid particles in the dressing material is 20-80% by weight based on the total weight of the dressing material.

Further, the composite dressing material is capable of accelerating softening or liquefying necrotic tissue or eschar on the wound, thereby resulting in autolytic debridement of the wound within 1-4 days.

In another embodiment, the composite dressing material is composed of at least two separate layers, wherein the first layer comprises a porous, polymeric substrate impregnated with a semi-solid hydrophobic composition, and the second layer comprises a polymeric substrate comprising a hydrophilic composition.

According to this embodiment, the hydrophilic composition is preferably a composition comprising a hydrocolloid such as modified cellulose such as carboxymethyl cellulose and hydroxyethyl cellulose, or alginate, chitin, chitosan, and hyaluronan.

In yet another embodiment, the composite dressing material is composed of at least two separate layers, wherein the first layer comprises a porous, polymeric substrate impregnated with a semi-solid hydrophobic composition, and the second layer comprises a porous, foam polymeric substrate.

In yet another aspect of the present invention, a kit of skin or wound composite dressing is provided, comprising said composite dressing material and an outer packaging material enclosing the dressing material.

Further, the kit also comprises a protective sheet covering the wound-facing-side of the active layer of the composite dressing material, and/or an adhesive material for securing the dressing material to the skin of the mammal.

In a further aspect, the present invention provides a method of manufacture of the composite dressing material of the present invention, comprising the steps of: impregnating a porous, polymeric substrate with a semi-solid hydrophobic composition comprising fatty acid and solid inorganic particles such that the solid inorganic particles are substantially evenly embedded in the matrix of the polymeric substrate.

Further, in an embodiment, the method of manufacture comprises the step of grinding the solid inorganic particles; and adding the ground particles to the fatty acid; heating and mixing well so as to produce a hydrophobic composition that is semi-solid at room temperature; heating and liquefying the semi-solid hydrophobic composition; and substantially evenly spreading the liquefied hydrophobic composition onto one or more sheets of the porous, polymeric substrate, producing the composite dressing material upon cooling to room temperature.

In another aspect of the present invention, a method is provided for treating a wound of a mammal, which comprises: positioning a composite dressing material in contact with the wound site and securing the dressing material to the skin of the mammal.

The type of wound includes, but not limited to, acute surgical and traumatic wounds, bums (such as thermal, electrical bums, radiation, chemical, frost, and wind chill bums, as well as sunburns), diabetic ulcers, venous ulcers, arterial ulcers, pressure ulcers (otherwise known as decubitus ulcers or bedsores), skin ulcers of mixed etiologies such as ulcers caused by two or more of the disease selected from the group consisting of diabetes, cardiovascular disease, peripheral vascular disease, vasculitis, central or peripheral neuropathy, renal disease, autoimmune disease, and cancer, fistulas, skin fissures (caused by eczema, contact dermatitis, psoriasis, folliculitis, acne, lupus, herpes, etc.), and other chronic or necrotic wounds and inflammatory lesions and disorders. The materials according to the present invention are primarily intended for the treatment of both infected wounds and non-infected wounds (that is to say wounds showing no clinical signs of infection). The type of wound is not limited to an open wound of the skin, and may include intact skin but with inflammation or damage of the skin or the tissue underneath the skin, such as skin inflammation, fibrosis, dermatitis, erythema or edema due to irradiation (such as cancer radiotherapy). The dressing material can also be used prophylactically to treat skin damage by positioning the dressing on the target skin area to be affected, and then treating the target area with an energy therapy, so as to prevent tissue inflammation, fibrosis or other forms of injury caused by the energy therapy.

In the course of the treatment, the dressing material can be used as a primary dressing in direct contact with the wound bed throughout the whole treatment process, or in combination with other wound treatment modalities such as bioactive cell-based wound dressings, autologous or allogeneic skin grafts (including xenografts), acellular matrix, amniotic membrane, negative pressure wound therapy, and hyperbaric oxygen therapy.

The innovative dressing materials of the present invention are robust, easy to use with one piece of dressing changed each time, non-adherent to the wound, and without complex aid of adjunct machines and technical training. The dressings can be broadly applied in the settings of in-patient and out-patient treatment, rehabilitation facilities, nursing homes, home health care, as well as under extreme circumstances such as rescue in the wildness and battle fields. Owing to their superb therapeutic efficacy and healing capabilities, the present invention is particularly applicable to high-end specialized wound healing centers, diabetes management centers, specialty burn centers, plastic surgery, dermatology, and medical cosmetology.

### Brief Description of the Figures

These and other features and advantages of the invention will now be described with reference to the drawings of certain preferred embodiments, which are intended to illustrate and not to limit the invention, and wherein like reference numbers refer to like components, and in which:
Figure 1 is a schematic illustration of a thin-layer embodiment of the composite dressing material according to the present invention. A: A main view of the illustrated configuration; B: Cross-Dimensional illustration; C: A structural illustration of the fiber bundles in Figure 1A and 1B;
Figure 2 shows microscopic structures of an embodiment of the composite dressing material of the present invention. A: the net structure of the porous, polymeric substrate on a scale of 50 µm; B: microfilaments of the fiber bundles in the porous, polymeric substrate;
Figure 3 shows treatment of a patient with burn wounds with an embodiment of the inventive dressing material;
Figure 4 shows treatment of a patient with burn wounds with an embodiment of the inventive dressing material;
Figure 5 shows treatment of a patient with scalds by boiling water with an embodiment of the inventive dressing material;
Figure 6 shows treatment of a patient with scalds by boiling water with an embodiment of the inventive dressing material;
Figure 7 shows treatment of a patient with burn wounds with an embodiment of the inventive dressing material;
Figure 8 shows the microscopic structures of one type of the solid inorganic particles of an embodiment of the composite dressing material of the present invention under an electron scanning microscope (SEM). A: the microscopic structures of the solid inorganic particles on a scale of 20 µm; B: the microscopic structures of the solid inorganic particles on a scale of 3µm;
Figure 9 shows the microscopic structures of another type of the solid inorganic particles of an embodiment of the composite dressing material of the present invention under an electron scanning microscope (SEM). A: the microscopic structures of the solid inorganic particles on a scale of 20 µm; B: the microscopic structures of the solid inorganic particles on a scale of 2µm;
Figure 10 shows treatment of a patient with chronic wounds with an embodiment of the inventive dressing material;
Figure 11 shows treatment of a patient with burn wounds with an embodiment of the inventive dressing material;
Figure 12 (a contrasting case) shows the quality of healing of a patient with burn wounds treated with a burn ointment available on the market;
Figure 13 shows treatment of a patient with mechanically injured wounds with an embodiment of the inventive dressing material;
Figure 14 (a contrasting case) shows the quality of healing of a patient with mechanically injured wounds, similar to that case in Figure 13, treated with another wound therapy;
Figure 15 shows treatment of a patient with burn wounds with an embodiment of the inventive dressing material;
Figure 16 shows treatment of a patient with burn wounds with an embodiment of the inventive dressing material;
Figure 17 shows treatment of a patient with burn wounds with an embodiment of the inventive dressing material;
Figure 18 shows treatment of a patient with chemical burn wounds with an embodiment of the inventive dressing material;
Figure 19 shows treatment of a patient with charcoal burn wounds with an embodiment of the inventive dressing material;
Figure 20 shows treatment of a patient with burn wounds with an embodiment of the inventive dressing material;
Figure 21 shows treatment of a patient with burn wounds with an embodiment of the inventive dressing material;
Figure 22 shows treatment of a patient with burn wounds with an embodiment of the inventive dressing material;
Figure 23 shows treatment of a patient with electrical burn wounds with an embodiment of the inventive dressing material;
Figure 24 shows treatment of a patient with electrical burn wounds with an embodiment of the inventive dressing material;
Figure 25 shows treatment of a patient with electrical burn wounds with an embodiment of the inventive dressing material;
Figure 26 shows treatment of a patient with venous leg ulcers with an embodiment of the inventive dressing material;
Figure 27 shows treatment of a patient with venous leg ulcers with an embodiment of the inventive dressing material;
Figure 28 shows treatment of a patient with a non-healing surgical wound with an embodiment of the inventive dressing material;
Figure 29 shows treatment of a patient with a non-healing surgical wound with an embodiment of the inventive dressing material;
Figure 30 shows treatment of a patient with a non-healing surgical wound with an embodiment of the inventive dressing material; and
Figure 31 shows treatment of a patient with a foot gangrene with an embodiment of the inventive dressing material.

### Detailed Description of the Invention

The present invention provides innovative dressing materials, methods of use them, and kits or devices containing thereof.

Although there have been many variety of wound care products or devices developed to tackle specific issues of multi-phase wound healing process. For chronic wounds, the healing process can be even more complex involving many cellular players and environmental elements. The wound dressings commonly used in the clinic have some advantages, as well as some disadvantages such as 1) inability to prevent microbial invasion (e.g., cell-based skin substitutes or skin grafts, or acellular matrices); 2) leading to trauma to patients at the time of removal as they adhere to the wound surface (e.g., regular gauze or some silver-containing dressings that dry up at high body temperature of burn patients); 3) low absorption of wound exudates leading to accumulation of exudates at wound surface which then become site for microbial attack; 4) not providing proper permeability of gases; 5) can only be used for minor wounds and not for chronic wounds; and 6) being too dry or too wet-cannot maintain a physiologically moisture-balanced healing environment. Due to these disadvantages, multiple different wound management modalities have to be employed to resolve the complex issues in the progressive phases of the wound healing process.

The dressing materials provided by the present invention are uniquely innovative, multi-functional, high performance new materials that can be used as "one-stop" primary wound dressings throughout the wound healing process to painlessly, non-invasively repair and regenerate various wounds. In preferred embodiments, the dressing material uniquely comprises micro-particles that have tiny cavities or slits of dimensions in a nanometer scale and are organically integrated into the network of sub-micro filaments to mimic the skin barrier function: being capable of shielding contamination, moisture-absorption, moisture-retention, breathability, pliability and adhesion. The dressing materials are capable of not only absorbing wound exudates to facilitate rapid, non-invasive autolytic debridement and inward/outward regulation to ensure thorough drainage, but also targetedly and compartmentally retaining exudates in the dressing so as to maintain a semi-occlusive local environment with a physiological moisture balance, which is conducive to intercellular and intracellular signal transduction, promoting migration of autologous fibroblasts and epithelial cells, regeneration of granulation tissue and epithelium, accelerating physiological healing and restoration of wounds, avoiding or minimizing skin grafting so as to reduce the pain and scarring of patients.

The dressing materials provided in the present invention are versatile and can be used to heal a wide variety of acute and chronic open or closed wounds, throughout all phases of the wound healing process: coagulation; inflammation; cell proliferation and repair of the matrix; epithelialization and remodeling of scar tissue. The composite dressing material is preferably in sheet form, and can be packaged in a one-time ready-to-use form, a feature very desirable for busy clinicians who wish not to be bothered with the preparation (e.g., thawing of the frozen cell-based skin substitutes, washing them and maintaining at a narrow temperature range for viability of the cells) or pretreatment (e.g., wetting of dry dressing containing therapeutic powder) of the dressing materials.

The present invention is also cost effective at every level. As the dressing can take a conveniently packaged sheet form stored at room temperature, clinician, care takers and/or patients themselves can manage the wounds without going through elaborate technical training or handling any instruments with minimal pain and optimal healing times. The dressing is cost effective for institutional healthcare providers (e.g., hospitals, clinics, wound care centers, burn centers, skilled nursing facilities, rehabilitation facilities, long term care facilities, senior care or assisted living centers) may only need to inventory this inventive dressing as a primary dressing for managing many different types of wounds and skin lesions, acute or chronic.

The inventive dressing material is a composite material possessing the properties and attributes of an effective skin substitute to protect the skin or wound bed: as a thin layer of proper thickness and embedded with oily semi-solid to closely adhere and naturally conform to the contours of a wound bed to form a semi-occlusive physical barrier mimicking the skin barrier, effectively block contamination of environmental pathogens, while creating a buffered environment to retain warmth and moisture to effectively cover and protect the wound so as to minimize eschar formation due to rapid evaporation of a large amount of moisture from the wound.

The inventive dressing material is soft and pliable, being capable of conforming to the contours of a wound bed, an ability that the inventors believe to be critical to effectively controlling microbial infection and inflammation. Compared with many types of wound dressings on the market, the inventive dressing material could be gently pressed to "micro-contour" to a wound bed so as to minimize voids and spaces where bacteria can thrive, thereby reducing the risk of bacterial infection and formation of antibiotic-resistant biofilms. At the level of clinical application, as the inventive dressing material is highly pliable, oily moist and elastic, it closely adheres and naturally conforms to various physiological contours of wound beds, which would prevent dislodging of the dressing when the patient is moving around. At the same time, the dressing is highly conformable to the body's contours and comfortably wearable, the dressing time can be extended to protect the wound longer so as to reduce the frequency of dressing change, a feature very much welcome by the healthcare professionals and patients.

Upon removal from the wound bed, the inventive dressing material is non-adherent to wound bed and does not disturb the kinetics of healing or cause pain to the patient. It also functions as a semi-occlusive and breathable skin barrier to create an ideal moisture-balanced microenvironment conducive to regenerative wound healing-not too wet so as to cause maceration and not too dry so as to cause desiccation of the nascent regenerated healthy tissue. The dressing materials can be stored at room temperature for at least 12 months and ready to be used to dress a wound without any pretreatment.

As demonstrated in the clinical cases disclosed herein, the dressing material is also multifunctional-being capable of autolyticly debriding non-viable or necrotic tissue to control microbial infection (e.g., without resorting the painful sharp surgical debridement of necrotic tissue), providing an anti-oxidative relief to promote cell proliferation, and stimulating microcirculation to promote tissue regeneration, including capillary and nerve regeneration. As described in the EXAMPLES section, such dressing materials have been clinically demonstrated to accelerate healing of acute and chronic wounds with superior healing quality and minimal scarring.

In one aspect of the present invention, a composite skin or wound dressing material is provided, which comprises a layer of a porous, polymeric substrate impregnated with a semi-solid hydrophobic composition, wherein the composition comprises fatty acid and fine solid particles embedded in the matrix of the polymeric substrate.

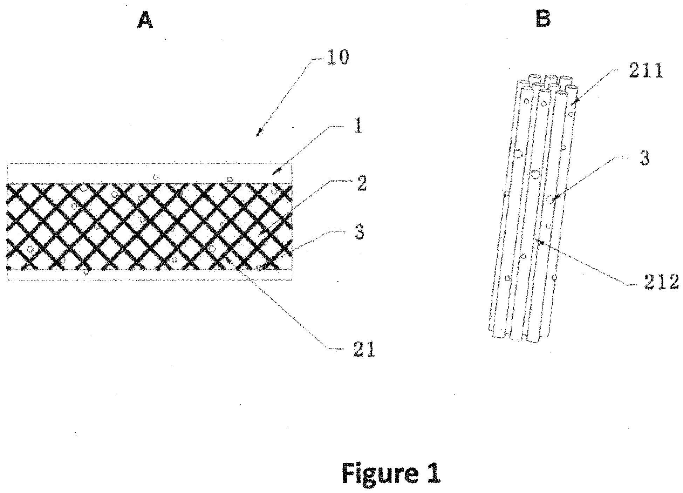

Figure 1 is a schematic illustration of a thin-layer embodiment of the composite dressing material according to the present invention. Figure 1A shows a top-down view of the composite dressing material laid horizontally on a surface. As illustrated in Figure 1A, the composite dressing material comprises an active layer 10 wherein there is a porous, polymeric substrate 2 with a net structure. According to the invention, the net structure of the porous, polymeric substrate 2 can be regular or irregular.

According to this embodiment, the material that the net structure of the porous, polymeric substrate 2 is composed of fiber bundles 21.

In the active layer 10 there are solid inorganic particles 3 embedded in the porous, polymeric substrate 2 with the particles 3 substantially evenly distributed in the substrate 2. As Figure 1A is a schematic illustration that merely reflects the geological distribution of the solid inorganic particles 3, not the actual size or dimension of the components. Also shown in Figure 1A is that fatty acid 1 soaks through the porous, polymeric substrate 2.

Figure 1B is a cross-dimensional illustration of the thin layer embodiment of Figure 1A. As the thickness of the thin layer dressing material is quite small, only 0.1 -10 mm, to clearly illustrate its structure, the illustration has been magnified. Figure 1B merely reflects the basic configuration of the dressing material, not its actual size or dimension. As illustrated in Figure 1A and 1B, fatty acid 1 substantially evenly soaks through the porous, polymeric substrate 2, forming a substantially even layer while fatty acid 1 can overflow to the upper and lower surfaces of the porous, polymeric substrate 2. According to the embodiment, on the surface of the porous, polymeric substrate 2 to be directly mechanically processed, the thickness of the fatty acid 1 can be larger than that on the surface of the substrate on the opposing side.

Figure 1C is a structural illustration of the fiber bundles 21 in Figure 1A and 1B. As illustrated in Figure 1C, each of the fiber bundles 21 is composed of multiple microfilaments211.There is interstitial space 212 among the multiple microfilaments 211. The solid inorganic particles 3 substantially evenly distribute on the surface of the microfilaments 211.

In the embodiment of the present invention, the longest cross diameter of the microfilament is 0.01-20 µm, preferably 0.01-10 µm or 0.01-6 µm. The interstitial space among the multiple microfilaments is preferably to 0.01-10 µm or 0.01-3 µm.

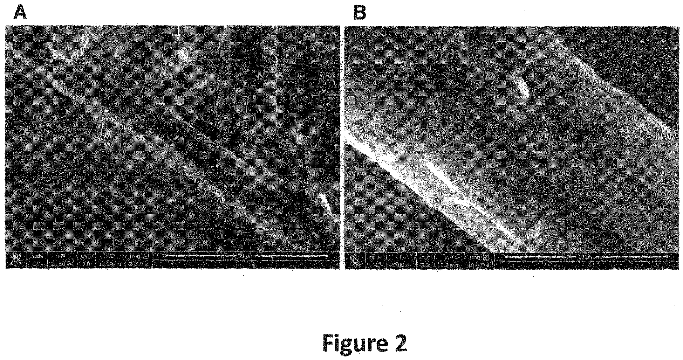

Figures 2A and 2B show microscopic structures of an embodiment of the composite dressing material of the present invention. Figure 2A shows the net structure of the porous, polymeric substrate on a scale of 50 µm, as well as the substrate impregnated with the fatty acid and the solid inorganic particles embedded in the polymeric substrate. Figure 2B shows microfilaments of the fiber bundles of the porous, polymeric substrate, bamboo viscose, on a scale of 10 µm, as well as the solid inorganic particles attached to the microfilament of fiber bundles. Also shown is the interstitial spaces, large or small, among the microfilaments, much like capillaries that can effectively absorb and evaporate moisture. The different orientations of the microfilaments create interstitial spaces on a nano- and micro-meter scale. Such a unique structure enables the dressing to acquire excellent breathability, moisture-retention capability and permeability.

Through the capillary effect of the microfilaments with sub-micro interstitial space-absorbing and slowly "breathing out"/ releasing moisture via the interstitial spaces and micro-grooves on the surface of the microfilaments, the composite dressing material of the present invention can prevent maceration of the wound that impedes skin regeneration. Such a dual directional adjustment, moisture retention and release, maintains a physiological balance of moisture, thereby creating an ideal local environment for wound healing.

Such a dressing on the wound creates a semi-occlusive local environment with a physiological balance of moisture, which effectively promotes selectively autolytic embodiment of necrotic tissue and eschar without harming the peri-wound skin. The dressing can lock in wound exudates to soften the necrotic tissue while the exudates release and activate various enzymes and activating factors (especially proteinases and kinases) to promote lysis of the fibroprotein and necrotic tissue. The exudates also contain phagocytes and neutrophils that produce lytic substances to effective lyse necrotic tissue. Debris of the lysed necrotic tissue can be removed from the wound bed upon dressing change, thereby avoiding or minimizing the use of low-selective, painful surgical debridement.

The inventors have surprisingly observed that the inventive dressing material can rapidly soften and liquefy necrotic tissue and eschar to enable autolytic debridement with 1-3 days. Upon dressing change debris of the lysed necrotic tissue can be removed from the wound bed to facilitate a non-invasive autolytic debridement without damaging the healthy skin surrounding the wound, thereby reducing inflammation, promoting rapid regeneration of granulation tissue. In contrast, dressings often used in the prior art to promote autolytic debridement, such as hydrogels and hydrocolloids, need at least 6-7 days. Collagenases used to enzymatically debride suffer great limitations in clinical application due to their poor selectivity between necrotic tissue and healthy tissue of the peri-wound; and their high prices as biochemical enzymatic agents also create big burdens on the patients and the healthcare system.

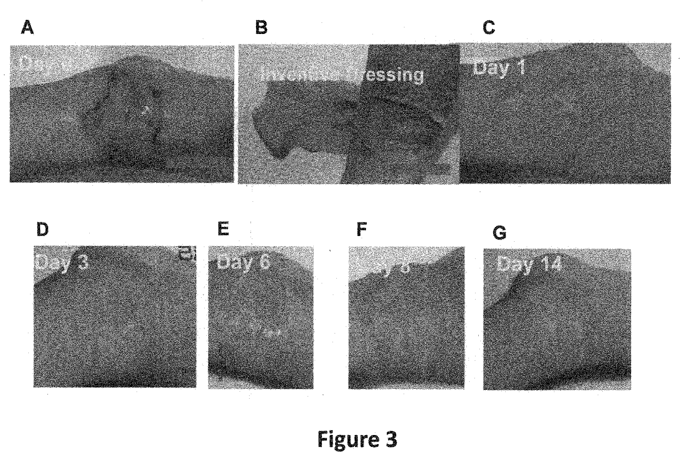

Figure 3 shows treatment of a patient with burn wounds using the dressing material of the present invention: male, age 10; sustained deep 2^{nd} degree scalds by boiling water on the left ankle; wound condition worsened in a prior treatment for 4 days using a burn ointment; then treated with the inventive dressing; dressing change every 1-2 days. Figure 3A shows that prior to the inventive treatment there was a large amount of necrotic tissue and eschar with erythema and inflammation of the wounds and the periwound areas. After one day of treatment using an embodiment of the inventive dressing, the eschar on the wounds substantially liquefied through autolytic debridement (Figure 3B). Upon cleansing of the wounds using saline, the wounds were clean and left with a thin layer of crust (Figure 3C). Inflammation of the wounds and the periwound areas significantly subsided. After 14 days of treatment, the wounds healed completely without obvious scarring (Figures 3D, E, F, G).

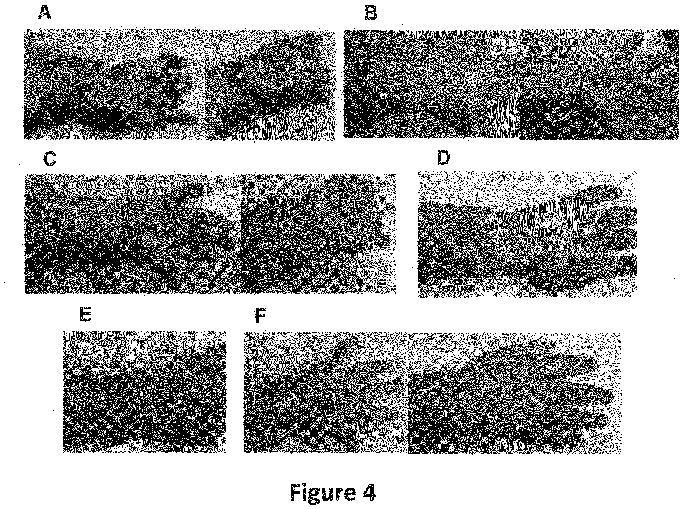

Figure 4 shows treatment of a patient with burn wounds using the dressing material of the present invention: male, age 1 year and 5 months; sustained deep 2^{nd} and 3^{rd} degree scalds by boiling water on the right arm and hand; wound condition worsened in a prior treatment for 16 days using a burn ointment (and also due to exposure of wounds in the air in winter time resulting in tissue necrosis); high fever for several days; then treated with the inventive dressing; dressing change every 1-2 days. Figure 4A shows that prior to the inventive treatment there was a large amount of necrotic tissue and hard eschar with erythema and inflammation of the wounds and the periwound areas, coupled with systemic inflammatory reactions. After one day of local treatment using an embodiment of the inventive dressing, coupled with systemic treatment with antibiotics and anti-inflammatory drugs, the hard eschar on the wounds substantially liquefied through autolytic debridement; upon cleansing of the wounds using saline, the wounds were clean (Figure 4B) with fresh granulation tissue appearing after 4 days of treatment (Figure 4C). Inflammation of the wounds and the periwound areas significantly subsided. After 46 days of treatment, the wounds healed completely without skin grafting (Figures 4D, E, F).

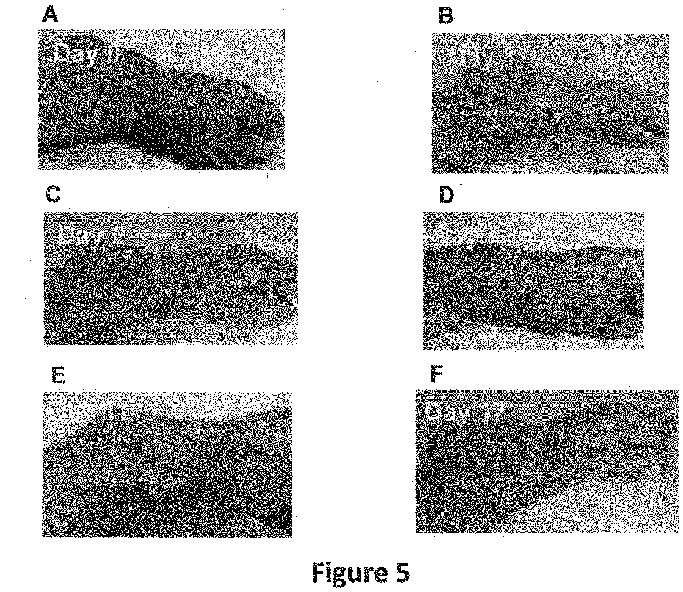

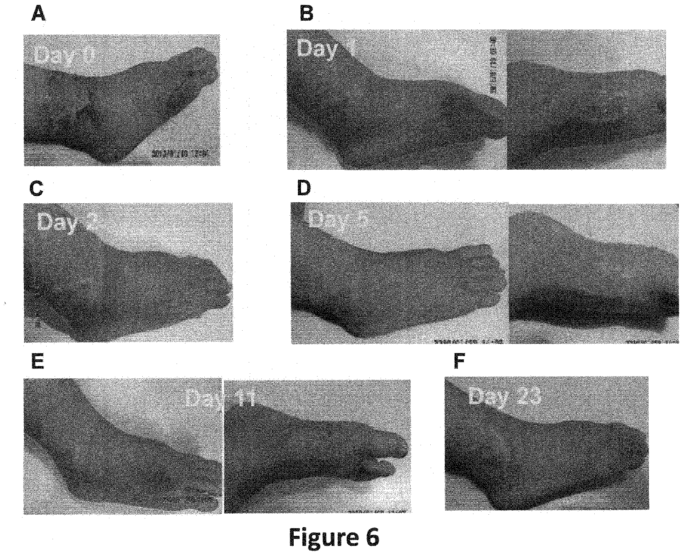

Figures 5 and 6 show treatment of a mother and her son, both simultaneously sustained deep scalds by boiling water, by using the dressing material of the present invention: The mother, age 32; sustained deep scalds on the right foot and ankle; wound condition worsened to become 3^{rd} degree wounds in a prior treatment for 18 days using two kinds of burn ointment; then treated with the inventive dressing; dressing change every 1-2 days. Figure 5A that shows prior to the inventive treatment there was a large amount of necrotic tissue, eschars and ulcers beneath thereof, with erythema and inflammation of the wound and the periwound area. After one day of local treatment using an embodiment of the inventive dressing, the eschars on the wounds substantially softened and liquefied; upon cleansing of the wounds using saline and removal of the softened eschars, the wounds were clean (Figure 5B) with fresh granulation tissue appearing; and inflammation of the wounds and the periwound areas significantly subsided. After 17 days of treatment, the wounds healed completely without obvious scarring (Figures 5C, D, E, F). The son, age 3; sustained deep scalds on the right foot and ankle to a similar degree of his mother and went through the same prior treatment as his mother; wound condition worsened to become 3^{rd} degree wounds using two kinds of burn ointment (Figure 6A); then treated with the inventive dressing following a similar process. After one day of local treatment using an embodiment of the inventive dressing, the eschars on the wounds substantially liquefied via autolytic debridement; upon cleansing of the wounds using saline, the wounds were clean (Figure 6B). After 23 days of treatment, the wounds healed completely without obvious scarring (Figures 6C, D, E, F).

The composite dressing materials of the present invention are highly absorbent, being capable of effectively absorbing exudates, sloughs, debris of necrotic tissue as a result of autolytic debridement, which, upon dressing change, were removed, thereby facilitating effective drainage so as to prevent infection and inflammation of the wound and reducing exudation.

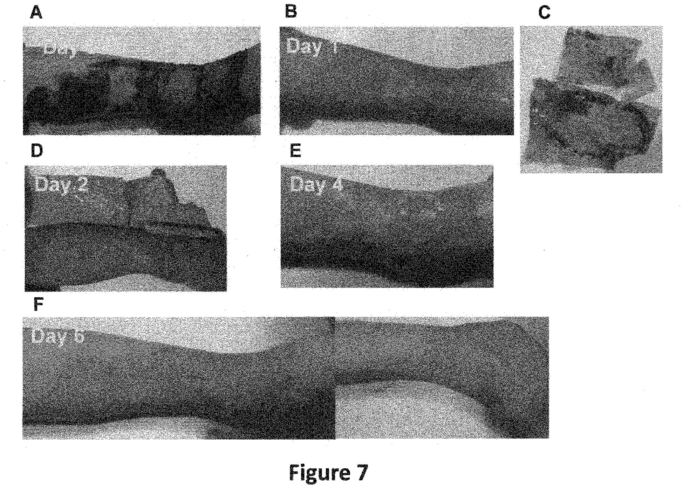

Shown in Figure 7 is treatment of a patient with burn wounds using an embodiment of the invention: male, age 4; sustained deep 2^{nd} degree scalds by boiling water on the left lower leg and foot; wound condition worsened in a prior treatment for 15 days using another burn therapy; then treated with the inventive dressing; dressing change every 1-2 days. Figure 7A shows that prior to the inventive treatment there was a large amount of necrotic tissue and black eschars with erythema and inflammation of the wounds and the periwound areas. After one day of treatment using an embodiment of the inventive dressing, some eschars on the wounds liquefied through autolytic debridement while the rest of the unliquefied black eschars were removed upon change of the dressings (Figure 7C). The wounds were left very clean (Figure 7B); and inflammation of the wounds and the periwound area significantly subsided. After 6 days of treatment, 70% of the wounds healed (Figures 7D, E, F).

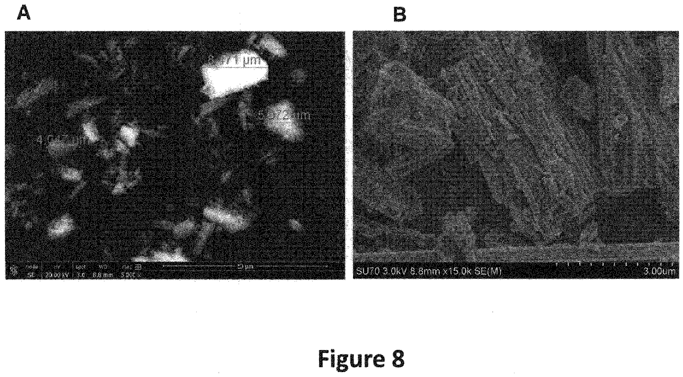

Figures 8A and 8B show the microscopic structures of one type of the solid inorganic particles of an embodiment of the composite dressing material of the present invention under an electron scanning microscope (SEM). Figure 8A shows the microscopic structures of the solid inorganic particles on a scale of 20 µm, presenting as micro-particles of about 1-10 µm in length. Figure 8B shows the microscopic structures of the solid inorganic particles on a scale of 3 µm, demonstrating that there are micro-grooves on the surface of the column-shaped micro-particles, with the size of the groove distributing in about 10-500 nm.

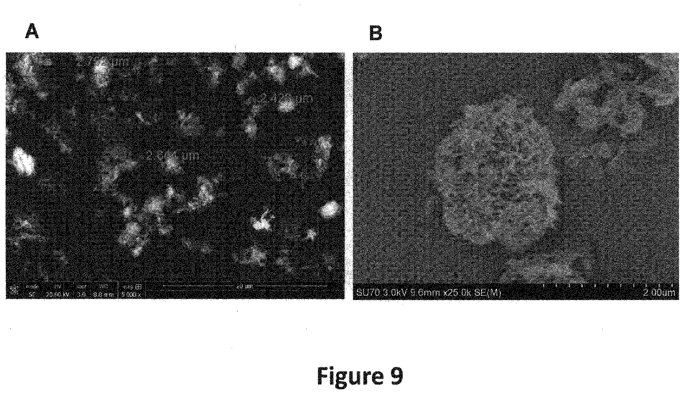

Figures 9A and 9B show the microscopic structures of another type of the solid inorganic particles of an embodiment of the composite dressing material of the present invention under an electron scanning microscope (SEM). Figure 9A shows the microscopic structures of the solid inorganic particles on a scale of 20 µm, presenting as micro-particles of about 1-10 µm in length. Figure 9B shows the microscopic structures of the solid inorganic particles on a scale of 2 µm, presenting the particles as thin-flaked clusters with wedge-like cavities formed by clustering of the thin flakes, wherein the cavities have a size distributed in about 10-300 nm.

Preferably, the average size of the solid inorganic particles of the inventive composite dressing material is preferably 0.01 - 50 µm. More preferably, at least 80% of the solid inorganic particles has the size of less than 50, 30, 20 or 10 µm.

Generally, nanomaterials are referred to those materials having a basic 3-dimensional structure with at least one dimension within the range of nanometers (1-100 nm), possessing extremely large specific surface area (SSA) and high porosity. It is generally recognized that nanomaterials have properties of quantum size effect, small -size effect, surface-interface interactive effect and synergistic effect, demonstrating as a large specific surface area, many surface active centers, high activity of surface reactions, strong absorbability, high catalytic activity, etc.

The composite dressing materials of the present invention employ such above-described solid inorganic particles, thereby leveraging the unique advantages of nanomaterials to enhance the effective absorptive area on the surface. Such features, combined with the porous polymeric net with microfilaments, further increase adhesiveness and enhance the anti-microbial bioactivity of the dressing, so as to effectively absorb exudates, sloughs, debris of necrotic tissue as a result of autolytic debridement, which, upon dressing change, were removed, thereby facilitating effective drainage so as to prevent infection and inflammation of the wound and reducing exudation.

In clinical practice, the inventors further discovered that the composite dressing materials also possess a property of targeted and compartmentalized wound protection. The dressing can protect the wound areas with different depths in a targeted and compartmentalized manner: the dressing can directionally absorb and lock in excessive exudates on the wound with deeper injury and more exudation, preventing their diffusion into the surrounding areas with shallower, less-exuding wounds, thereby protecting the shallower wounds from healing-impeding maceration.

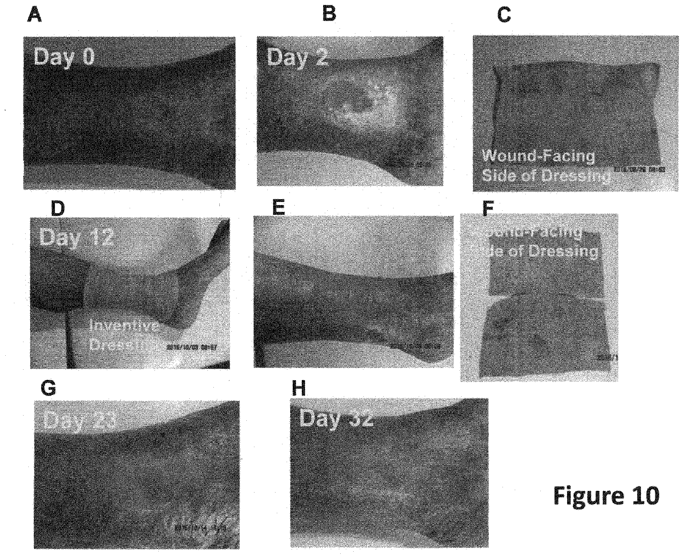

Shown in Figure 10 is treatment of a patient with chronic wounds using an embodiment of the invention: male, age 61; with a 10-year history of varicose veins; skin infected due to scratching 2 years ago, gradually developing enlarging and deepening non-healing chronic ulcers; ulcer condition worsened due to a recent collision rendering further enlargement of the wound area, resulting in painful claudication; ulcers non-healing by prior treatment; then treated with the inventive dressing; dressing change every 2-3 days. Figure 10A shows that prior to the inventive treatment there was a large amount of necrotic tissue, sloughs, fibrous eschar with erythema and hyperpigmentation of the periwound areas. After 2 days of treatment using an embodiment of the inventive dressing, eschars on the wounds liquefied through autolytic debridement and were removed upon change of the dressings (Figure 10C). The wounds were left very clean (Figure 10B); and inflammation of the wounds and the periwound area significantly subsided. Figure 10C shows that the liquefied necrotic tissue was compartmentally locked in the dressing area, and was prevented to diffuse into the surrounding areas with shallower, less-exuding wounds. Figure 10D shows that after 12 days of treatment, granulation tissue on wound bed appear to be fresh and alive, and the exudates infiltrated vertically and were absorbed by the dressing (Figure 10E). Figure 10F shows that on the dressing removed from the wound the exudates were compartmentally locked in the dressing, preventing them from diffusing into the surrounding peri-wound area. After 32 days of treatment, the wounds substantially healed (Figures 10G, H).

The inventors further discovered that the composite dressing materials of the present invention effectively mimics the skin barrier function, creating a semi-occlusive local environment with a physiological moisture balance, thereby greatly beneficial to the reduction of scarred healing of deep wounds. In such a physiologically moist environment (wherein hyaluronic acid arises faster with its concentration maintained for a longer time), fibroblasts migrate fast with a strong mobility while their proliferation synchronizes with the synthesis of collagen, thereby preventing disorderly and excessive deposition of collagen. Such mode of wound healing is similar to that of scarless wound healing in the fetus at early gestational stages.

The inventive composite dressing materials with inorganic microparticles integrated into the network made of microfilaments can timely and effectively absorb wound exudates, sloughs, debris of necrotic tissue post autolytic debridement, thereby reducing inflammation caused by cellular toxins and metabolites, and consequently preventing wound healing with deep and thick fibrotic scars as a result of over-production, disorderly and excessive deposition of type I collagen due to over-proliferation of fibroblasts. In particular, contracture scar is a type of scar categorized according to its impact on the body or organ's function, which not only impedes the function but also change the appearance. A deeper wound that encompasses a joint area, even after the wound heals, can easily result in contracture scarring due to the impact of continuous tension. Especially for the joints of the hands and feet, due to their intrinsic contraction and proliferation formation of flipper-like scars can result in limitation of stretchability, mobility and range of motion, as well as distortion and dislocation.

In clinical practice, the inventors have observed that the inventive composite dressing materials can not only rapidly heal deep wounds on the joint areas (such as deep 2^{nd} and 3^{rd} degree bums), but also effectively prevent formation of contracture scars, thereby greatly minimizing dysfunction, restoring physiological function and appearance of the wound site. Such advantageous features are of great significance in the areas of plastic surgery and medical cosmetology as the pain and burdens of medical expenses of multiple scar reconstructive surgeries can avoided.

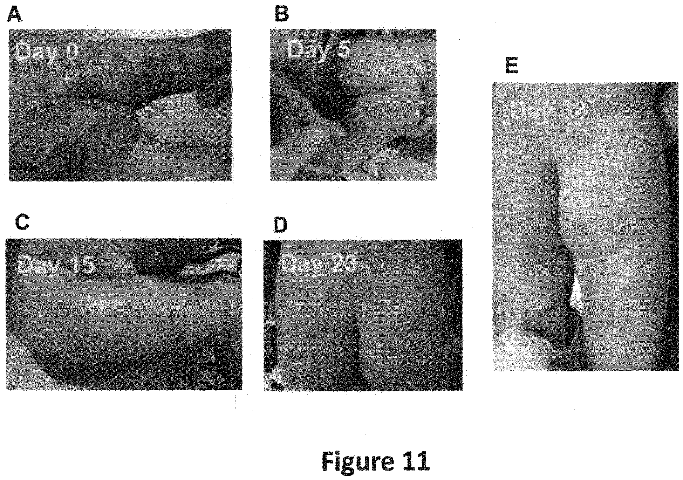

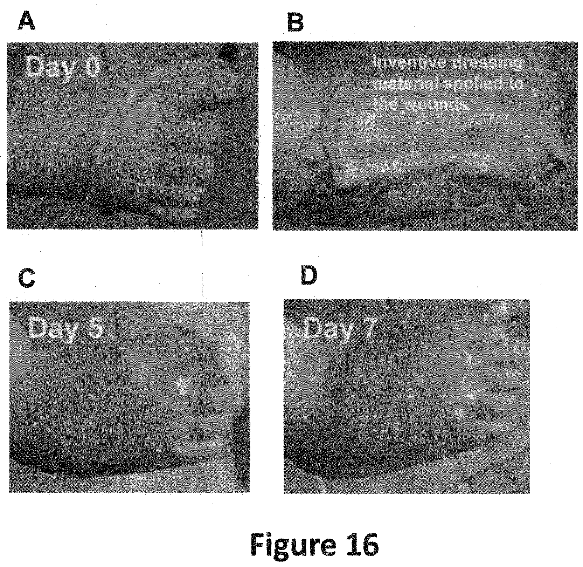

Figures 4, 19, 20, 23, 24 and 25 show the patients with deep burn wounds on the hands treated with an embodiment of the inventive dressing materials. Figures 3, 5, 6, 7 13 and 14 show the patients with deep burn wounds on the ankles treated with an embodiment of the inventive dressing materials. Figures 16, 18, 30 and 31 show the patients with deep burn wounds on the feet treated with an embodiment of the inventive dressing materials. Figures 11, 21 and 22 show the patients with deep burn wounds on the kneels treated with an embodiment of the inventive dressing materials. It can be seen that upon healing of the deep wounds, there was no formation of contracture scars, nascent tissue, including the skin, is supple and flat, essentially scarless.

Not wishing to be bound to the theory, the inventors believe that the calcium and zinc-containing inorganic particles in the embodiment of the inventive composite dressing materials can regulate synthesis and degradation of collagen through matrix metalloproteinases (MMPs) to reduce scarring. MMPs are endogenous polypeptidases containing calcium and zinc. The presence of calcium and zinc is closely related to the collagen hydrolytic activities of MMPs. Compared with the normal skin, keloids and hypertrophic scars contain less calcium, zinc, copper, iron, manganese, and selenium. With the MMP activity reduced, collagen synthesis exceeds degradation, leading to hypertrophic scarring. Calcium and zinc in the inventive composite dressing materials could effectively regulate MMP-1 to degrade excessive type I collagen, as well as inhibit cellular activity of fibroblasts through the calcium channels on the cell surface so as to reduce the excessive production of type I collagen.

The composite dressing material is preferably in sheet form and comprises an active layer of the dressing material according to the invention. The active layer would normally be the wound contacting layer in use, but in some embodiments it could be separated from the wound by a separate layer of wound treatment agent, such as bioactive cell-based wound dressings, autologous or allogeneic skin grafts (including xenografts), acellular matrix, amniotic membrane, etc.

The area of the dressing sheet individually can be cut into any size or shape, with surface area being preferably 2-1600 cm², more preferably being 2-400 cm², and most preferable 4-100 cm². The thickness of the dressing sheet is preferably 0.1 - 10 mm, 0.2-5 mm, or 0.4-2 mm.

The dressing sheet can be sterilized by ionizing radiation such as (cobalt-60, caesium-137, etc.), or by chemical sterilization such as ethylene oxide (EtO) and nitrogen dioxide (NO₂) gas.

The polymeric substrate is preferably a liquid-absorbent but non-bioabsorbable polymer that is not fully degraded and absorbed *in vivo* in the mammalian body. Such polymers include natural fibres or synthetic polymers. The natural fibers include, but are not limited to, cotton, silk and linen. Synthetic polymers include, but are not limited to, fibers manufactured from chemically synthesized polymers such as polyester, nylon, polyamide, polypropylene, polyurethane, polytetrafluoroethylene, and fibers manufactured from naturally available polymers such as cellulose from wood pulp or bamboo pith to produce viscose/rayon/lyocell/TENCEL/MODAL (also known as "artificial silk" or "regenerated cellulose fibers and fabrics"), fibers originated from wood pulp (e.g., viscose, etc.) or bamboo (bamboo fiber, bamboo viscose, etc.), regenerated fibers of protein origin come from plant protein (such as corn, soy, alginate, and peanut) or from animal protein (such as casein from milk), alginate, chitin, chitosan, hyaluronan, etc. The fibres can be woven or non-woven.

The semi-solid hydrophobic composition is preferably substantially homogenous such that it is substantially evenly impregnated onto the matrix of the polymeric substrate to achieve a substantially homogenous layer of such an impregnated polymeric substrate at a thickness of about 01-10 mm, optionally 0.2-5 mm, 0.5-2 mm or 1-4 mm. The effect can be achieved by a precisely controlled mechanical manufacturing process, such as through adjusting the viscosity of the semi-solid hydrophobic composition, melting point (e.g., above 60°C, or above 70°C - 100°C, 80°C - 100°C), as well as adjusting the pressure of exerting the liquefied hydrophobic composition on the polymer substrate.

The composition is hydrophobic in that it contains less than 5%, preferably less than 1%, most preferably less than 0.2% of water by weight (based on the total weight of the dressing material) before it is positioned on the wound bed; and it contains 20-80%, optionally30-70% or 40-60%, by weight of fatty acid based on the total weight of the dressing material.

The fatty acid can be plant- or animal oil-based, preferably plant oil selected from the group consisting of soybean oil, rapeseed oil, olive oil, coconut oil, tea tree oil, sesame oil, pumpkin seed oil, corn oil, canola oil, castor oil, peanut oil, sunflower seed oil, cottonseed oil, chia seed oil, flax seed oil, safflower oil, broccoli seed oil, almond oil, tomato seed oil, pine nut oil, macadamia nut oil, camellia seed oil, jojoba oil, grape seed oil, rose hip oil, pomegranate seed oil, shea butter, evening primrose oil, lavender oil, and rosemary oil. Such oil can be a plasticizer assisting in providing a flexible, soft texture of the dressing. The plasticizer may also be mineral oil, petrolatum/petroleum jelly, silicon oil, stearates, hydrogenated ethers and esters, and a natural plant-based substitute for petrolatum such as shea butter, cocoa butter, unrefined coconut oil, tallow, lanolin, and jojoba oil.

The composition preferably contains a solidifier to solidify the oil and to render the composition semi-solid and pliable at room temperature. The solidifier is preferably beeswax (yellow or white), or emulsifying wax that is a combination of cetearyl alcohol, polysorbate (e.g., polysorbate60), polyethylene glycol (e.g., PEG-150) stearate, steareth (a synthetic polymer composed of PEG and stearyl alcohol such as steareth-20), etc.

The composition may further comprise suitable bioabsorbable polymers include those selected from the group consisting of collagens, elastin, bioabsorbable cellulose derivatives such as oxidized celluloses, galactomannans such as guar/borate, glycosaminoglycans such as cross-linked hyaluronates, polylactides/polyglycolides, polyhydroxybutyrates, and mixtures thereof. The preferred oxidized cellulose for practical applications is oxidized regenerated cellulose (ORC) prepared by oxidation of a regenerated cellulose, such as rayon, by exploiting the haemostatic properties of ORC that is advantageous in reducing post-surgical adhesion.

The oxidized regenerated cellulose (ORC) can be obtained by the process described in U.S. Pat. No. 3,122,479, the entire content of which is incorporated herein by reference. This material offers numerous advantages including the features that it is biocompatible, biodegradable, non-immunogenic and readily commercially available. ORC is available with varying degrees of oxidation and hence rates of degradation. The ORC may be used in the form of insoluble fibers, including woven, non-woven and knitted fabrics, or in the form of water-soluble low molecular weight fragments obtained by alkali hydrolysis of ORC.

The composition may further comprise a hydrocolloid to assist in adjusting the water absorbency and wicking properties of the dressing material of the present invention. Suitable hydrocolloids include alginates, pectin, gums such as guar gum or xanthan gum, modified celluloses such as carboxymethyl cellulose and hydroxyethyl cellulose, modified starches such as sodium starch glycolate, and mixtures thereof. Such hydrocolloid may be impregnated onto the matrix of the polymeric substrate of the inventive dressing material prior to the impregnation of a semi-solid hydrophobic composition or, alternatively, premixed with the semi-solid hydrophobic composition and then impregnated onto the matrix of the polymeric substrate.

In another embodiment, the composite dressing material is composed of at least two separate layers, wherein the first layer comprises a porous, polymeric substrate impregnated with a semi-solid hydrophobic composition, and the second layer comprises a polymeric substrate comprising a hydrophilic composition.

According to this embodiment, the hydrophilic composition is preferably a composition comprising a hydrocolloid such as modified cellulose such as carboxymethyl cellulose and hydroxyethyl cellulose, or alginate, chitin, chitosan, and hyaluronan.

In yet another embodiment, the composite dressing material is composed of at least two separate layers, wherein the first layer comprises a porous, polymeric substrate impregnated with a semi-solid hydrophobic composition, and the second layer comprises a porous, foam polymeric substrate.

The foam polymeric substrate may be a foam dressing comprising foam polymers such as those made from polyurethane, alginates, hydrocolloids, or polyacrylates.

The bilayer configuration can be very versatile in manufacturing different embodiments of the composite dressing material according to the present invention. For example, the dressing with the second layer comprising a hydrocolloid composition or a foam polymeric substrate can be used to dress those wounds with excessive exudates to absorb the fluid to the maximum capacity of the dressing so as to prevent leaking or maceration of the peri-wound area.

In yet another embodiment, the composite dressing material further comprises a backing sheet extending over the active layer of the porous, polymeric substrate impregnated with the semi-solid hydrophobic composition, the backing sheet positioned opposite to the wound facing side of the active layer. Preferably, the backing sheet is larger than the active layer such that a marginal region of width 0.2-10 cm, optionally 0.5-5 cm extends around the active layer to form a so-called island dressing. In such cases, the backing sheet is preferably coated with a pressure sensitive medical grade adhesive in at least its marginal region.

The backing sheet is preferably substantially liquid-impermeable, more preferably semipermeable that is permeable to water vapor, but not permeable to liquid water or wound exudate, so as to allow the wound under the dressing to heal under moist conditions without causing the skin surrounding the wound to macerate. Preferably, the backing sheet is also microorganism-impermeable. Examples of a suitable continuous conformable backing sheet include polyurethanes, polyalkoxyalkyl acrylates and methacrylates.

The solid particles embedded in the dressing material according to the present invention are preferably inorganic mineral particles, such as talc (magnesium silicate), silicon dioxide, magnesium oxide (MgO), aluminum oxide, zinc oxide (ZnO), iron oxides (FeO, Fe₂O₃, Fe₃O₄, Fe₄O₅, Fe₄O₃, etc.), calcium chloride, calcium carbonate (CaCO₃), zinc carbonate (ZnCO₃) calcium phosphate (Ca₃(PO₄)₂), calcium sulfate (CaSO₄),, and titanium dioxide (TiO₂).

The solid particles are preferably micronized, or ground and milled, into small, fine particles. The average size of the particles is preferably 0.01 - 50 µm, more preferably 0.05 - 20 µm, and most preferably 0.1 - 10 µm. The concentration of the solid particles in the dressing material is preferably 20-80%, optionally 30-70% or 40-60%, by weight based on the total weight of the dressing material.

The fine particles have small enough sizes so as not to inflict pain of the wound upon contact or frictions. As they are contained in the semi-solid hydrophobic composition and embedded in the matrix of the polymeric substrate, such a configuration could prevent rubbing the dry particles again the delicate wound bed. This semi-solid hydrophobic composition could also prevent granulation tissue from growing excessively into the dressing which could inflict pain upon dressing removal, damage the nascent regenerated tissues, and impede the wound healing process. As the particles are dispersed in the matrix of the polymeric substrate they create a reticulated, breathable matrix permeable for gas and water vapor, but not for microorganisms, thereby effectively protecting the wound bed from invasion of bacteria and other harmful microorganisms from the environment, as well as other environmental elements such as dust.

The diameter of a human cell is generally about 10-20 µm, and that of a cellular component ranges from several nanometers to several hundred nanometers. As such, nanoparticles can easily exchange information non-invasively with biomolecules intracellularly and extracellularly. Not wishing to be bound by the theory, the inventors believe that when impregnated on the matrix of the polymeric substrate, the solid particles, especially when smaller than 0.1 µm (100 nm), likely provide a biocompatible scaffold for tissue regeneration that more closely resembles the scale of the building blocks that form natural cellular support tissue such as support tissue for capillary endothelial cells to regenerate blood vessels, and neurons to regenerate nerve ends in the skin. The size of laminin, collagen, and fibronectin, which are major components of the neural extracellular matrix (ECM), is on the same order of magnitude, around 60 nm. These nanoparticles not only can mimic the natural tissue roughness, but also due to their unique surface energetic and interactions with proteins (via enhanced adsorption of select proteins and enhanced cell functions), can effectively promote tissue regeneration.

As shown in Figures 2, 8, 9, the microstructures of an embodiment of the inventive composite dressing material under a scanning electron microscope present that in the 3D structure the interstitial space among the microfilaments and the microgrooves on their surface have a size within the range of nanometers (<100 nm) in at least one dimension, possessing extremely large specific surface area (SSA) and high porosity, resembling the extracellular matrix (ECM), which is conducive to the attachment, differentiation and proliferation of cells. The microfilaments and inorganic particles in the dressing material can serve as an organizing structural scaffold, providing a suitable environment for attachment, differentiation and proliferation of cells, which is beneficial for tissue regeneration. Such an inventive material has a relatively large specific surface area, easily facilitating affixation of enzymes and their derivatives. On the other hand, its high porosity and connectivity between the cavities are all beneficial for affixing enzymes and biocatalysts on the wound, thereby maintaining higher survival rates.

The inventors further believe that by incorporating these fine solid particles into the matrix of polymeric substrate, the dressing material, upon application to the wound to conform to the contour of the wound bed (when mechanically deformed), the dressing materials can generate a transient electrical potential, the so-called a piezoelectric effect. As electrical stimulation as low as 10 mV/mm has been shown to be able to promote neuron growth, it is believed that such a piezoelectric material can effectively stimulate cellular activities on the wound bed, thereby enhance wound tissue regeneration, without any external electrical stimulation. The particles may also reduce the adhesion and density of bacteria on the wound bed, as well as disrupt the bacterial colony or biofilm formation, thereby reducing bioburden of the wound.

As shown in the clinical studies in the Examples section, the inventive dressing appears to possess the properties and attributes of an effective skin substitute- being soft, supple and flexible to conform to the contour of a wound bed in protecting the wound bed, and functioning as a semi-occlusive and breathable skin barrier to create an ideal moisture-balanced microenvironment conducive to regenerative wound healing, which is conducive to intercellular and intracellular signal transduction, promoting migration of autologous fibroblasts and epithelial cells, regeneration of granulation tissue and epithelium, accelerating physiological healing and restoration of wounds, avoiding or minimizing invasive skin grafting so as to reduce the pain and scarring of patients.

Figure 11 shows treatment of a patient with burn wounds using an embodiment of the inventive dressing material: female, age 3; sustained deep 2^{nd} degree scalds by boiling water on the rear waist, buttocks, and both lower limbs; wound condition worsened in a prior treatment for 1 day using a burn ointment; then treated with the inventive dressing; dressing change every 1-2 days. Figure 11A shows that prior to the inventive treatment there was a large amount of blister skin and necrotic tissue with erythema and inflammation of the wounds and the periwound areas. After 15 days of treatment using the inventive dressing, the blister skin and necrotic tissue on the wounds were debrided via autolytic debridement, and the wounds essentially healed (Figures 11B, C) with regeneration of dermis and epidermis without skin grafting. After 23 days of treatment, the wounds healed completely (Figure 11D). In a follow-up visit 38 days later it was observed that the healed skin resembles that healed via physiological regeneration without obvious scarring.

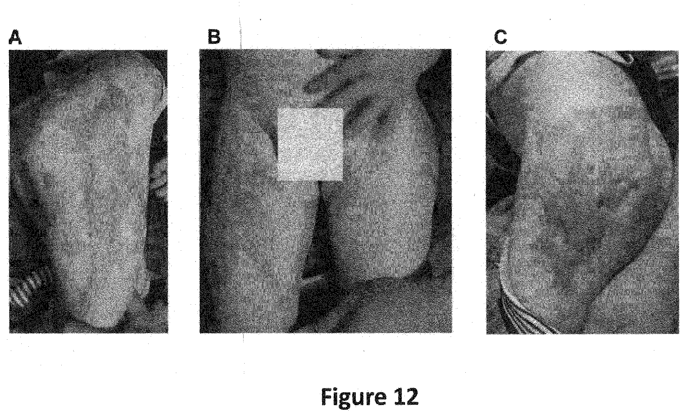

In contrast, the inventors have observed that treatment of pediatric patients with burn wounds to a similar degree using other kinds of dressings or topical drugs for bums could result in hypertrophic scarring. Figures 12A, B, and C show that a pediatric patient with deep 2^{nd} degree scalds by boiling water was treated using a burn ointment 10 months ago, and almost all of the wounds healed with hypertrophic scarring.

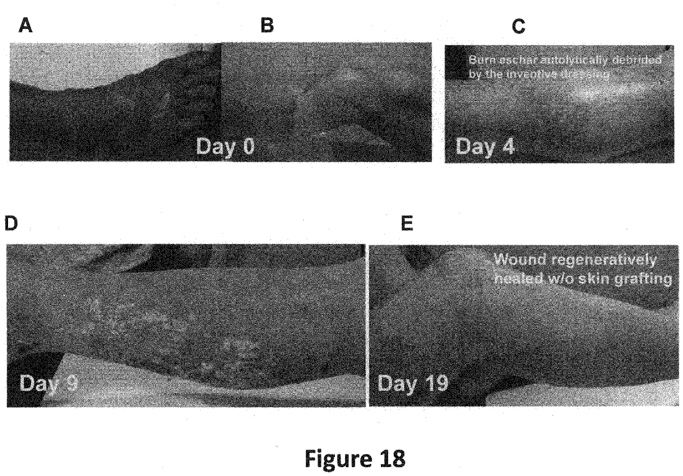

Figure 18 shows treatment of a patient with serious chemical bums using an embodiment of the inventive dressing material: male, age 58; right lower leg fell into a tank containing a high temperature (130°C/266°F) liquid mixture of lime, rescued a few minutes later and subsequently hospitalized in 3 hours; sustained a deep 2^{nd} and 3^{rd} degree bums (TBSA of 13%); standard systemic burn management employed; then treated with the inventive dressing; dressing change every 1-2 days. Figures 18A and 18B show that prior to the inventive treatment there was a large amount of blister skin and necrotic tissue. After 19 days of treatment using the inventive dressing, the blister skin and necrotic tissue on the wounds were debrided via autolytic debridement (Figure 18C); a large amount of granulation tissue grew and spread over the wound bed (Figure 18D); dermis and epidermis were rapidly regenerated; and the wounds essentially healed without skin grafting. Figure 18E shows that the healing is close to physiological healing without obvious scarring.

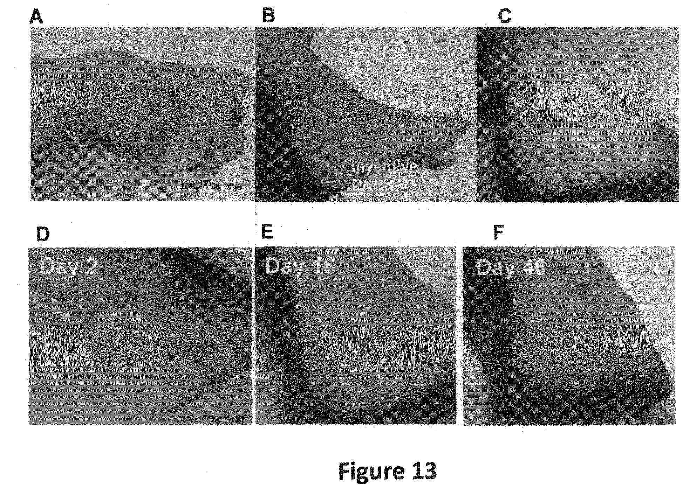

Figure 13 shows treatment of a patient with a mechanically injured wound using an embodiment of the inventive dressing material: female, age 6; her right heel sustained a muscle-reaching laceration by spokes of the wheel of a motorcycle; wound condition worsened after treatment with a wound ointment for 20 days; then treated with the inventive dressing; dressing change every 1-2 days. Figure 13A shows that prior to the inventive treatment there was a large amount of hard, fibrotic necrotic tissue with erythema and inflammation of the wounds and the periwound areas. The inventive dressing was applied to the wound (Figure 13B), and secured with a regular gauze bandage (Figure 13C). After 2 days of treatment using the inventive dressing, the hard eschars on the wound softened and liquefied through autolytic debridement and were removed upon dressing change, leaving a very clean wound (Figure 13D) with erythema and inflammation of the wound and the periwound area subsided. After 40 days of treatment, dermis and epidermis were regenerated; and the wounds essentially healed without skin grafting (Figures 13E, F). Figure 13F shows that the healing is close to physiological healing without obvious scarring.

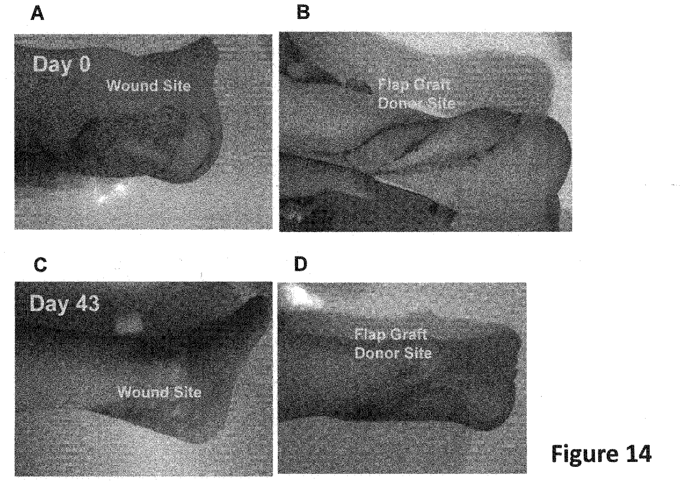

In contrast, the inventors have observed that treatment of patients with wounds to a similar degree on a similar site using other kinds of dressings or topical drugs for bums required skin grafting, which resulted in not only difficult healing but also hypertrophic scarring. Shown in Figure 14A and B are a pediatric patient (male, age 6, his right heel and lateral ankle sustained a muscle-reaching laceration by spokes of the wheel of a motorcycle) who was hospitalized for 28 days and went through bridement and flap grafting twice. After the grafting failed with the wound area expanded and deepened, the patient's family members rejected the hospital's suggestion of yet another round of flap grafting. After 43 days of treatment using the inventive dressing, the hard eschars on the wound softened and liquefied through autolytic debridement within 2-3 days and were removed upon dressing change. Dermis and epidermis were regenerated; and the wounds completely healed without skin grafting (Figures 14C) with improvement of skin condition on the donor site as well.

Not wishing to be bound by theory, the inventors believe that the fatty acid embedded in the composite dressing material, when applied to a live mammal and thus warmed up by the skin in the semi-occlusive, moist environment, the oil could permeate the wound surface and break up the necrotic tissues into particles by the process of saponification, thereby liquefying the necrotic tissue via the mechanism of autolytic debridement as quickly as within 1 week, sometimes within 1-3 days, 1-4 days or 3-5 days. The liquefied necrotic tissue can be easily removed with the removing of the inventive dressing (that absorbs the liquefied wound debris) upon dressing change, leaving the nascent regenerative wound bed clean and minimally disturbed. Compared to surgical debridement that may indiscriminately remove nonviable and health cells, this mode of autolytic debridement is non-invasive and maximally preserves the viability of the cells in the wound bed, including those cells that are in the stasis phase upon injury but could lose viability if not be revived timely. Thus, the depth of the wound can be controlled as shallow as possible, resulting in faster healing. By quick auto-debriding the necrotic or nonviable tissue, the impeded healing of the wound can be reactivated and kick-started to regenerate fresh granulation tissue and finally achieve wound closure.

The dressing material of the present invention may further comprise an antioxidant. Not wishing to be bound by theory, the inventors believe that prolonged oxidative stress may impede wound healing and tissue regeneration, as it can produce chronic inflammation, divert available energy supply towards antioxidant defense at the expense of tissue reconstruction, and increase levels of matrix metalloproteinases which cause granulation tissue breakdown. In chronic wounds, prolonged elevation of reactive oxygen species can lead to hydrogen peroxide-induced senescence or apoptosis or tissue necrosis, resulting in delayed wound regeneration.

Such an antioxidant or reactive oxygen scavenger may be a naturally occurring chemical or extract of natural materials, such as plants and animal components, preferably vitamin A, vitamin C, vitamin E, retinoids, ubiquinol, glutathione, carotenoids, green tea extract containing a polyphenol (e.g., catechin, epicatechin, gallocatechin, gallocatechingallate, gallocatechin, and epigallocatechin gallate (EGCG)), chlorophyllin, chlorophyllin, and water: ethanol extract of *Rubussuavissimus* (common name: Sweet Tea) leaf containing at least 50% polyphenol in the final extract powder. These natural materials may also exert certain anti-inflammatory and/or anti-allergic effects on the wound, which is particularly advantageous in healing chronic wounds.

The antioxidant or reactive oxygen scavenger may also be a synthetic antioxidant including, but not limited to, stilbenes, aniline dyes, acridine dyes, thionine dyes, such as gentian violet, aniline blue, methylene blue, crystal violet, acriflavine, indigo blue, brilliant green, trypan blue, trypan red, malachite green, zacrine, methyl violet, methyl orange, methyl yellow, ethyl violet, acid orange, acid yellow, acid blue, and acid red.

The antioxidant may be present in the dressing material of the present invention in an amount of from about 0.01% to about 10 % by weight, preferably 0.1 to about 5% based on the dry weight of the dressing material.

The dressing material of the present invention may further comprise an antimicrobial or antiseptic, to prevent or treat wound infection. The antimicrobial may be a naturally occurring or synthetic component, such as colloidal silver, silver salts, silver sulfadiazine, sucralfate, quaternary ammonium salts, antibiotics (e.g. tetracycline, penicillin, terramycins, erythromycin, bacitracin, neomycin, polymycin B, mupirocin, clindamycin and mixtures thereof), peptide antimicrobials (e.g. defensins, Magainin, synthetic derivatives thereof), chlorhexidine, povidone iodine, and triclosan. The naturally occurring antimicrobial can also be an extract of a botanical such as extract of *Paeoniasuffruticosa* (tree peony) bark, *Coptischinensis* (coptis) root, *Lithospermumerythrorhizon* (lithospermum) root, Rehmanniaglutinosa (rehmannia) root, Phellodendronchinense (cortex phellodendri) bark, or *Scutellariabaicalensis* root, which is preferably an oil extract; or an extract of *Houttuyniacordada* whole plant, *Lonicera japonica Thunb.* (Japanese Honeysuckle) flower extract, or *Isatisindigotica Fort.* (Indigowood) root or leave extract, which is preferably an ethanol: water extract.

The antimicrobial may be present in the dressing material of the present invention in an amount of from about 0.01% to about 10 % by weight, preferably 0.1 to about 5% based on the total weight of the dressing material.

The dressing material of the present invention may further comprise a pain reliever so as to reduce the pain of the wound. Such pain reliever may be naturally occurring or synthetic component, such as non-steroidal anti-inflammatory drugs (NSAIDs, such as ibuprofen, acetaminophen), anti-inflammatory steroids such as prostaglandins, codeine, hydrocodone, morphine, fentanyl, meperidine, methadone, oxycodone, and naloxone. The naturally occurring pain reliever can also be an extract of a botanical such as Corydalis yanhusuo (corydalis) tuber extract, borneol (extracted from plants such as *Cinnamomumcamphora,* or synthetic), or *Papaver somniferam L.* (poppy)dry capsules, which is preferably an oil extract.

The pain reliever may be present in the dressing material of the present invention in an amount of from about 0.001% to about 20 % by weight, preferably 0.01 to about 5% based on the total weight of the dressing material.

In another aspect of the present invention, a method is provided for treating a wound of a mammal, which comprises: positioning the composite dressing material of the present invention in contact with the wound site and securing the dressing material to the skin of the mammal.

The mammal could be a human or a mammal selected from the group consisting of dogs, cats, pigs, cows, bulls, horses, sheep, goats, tigers, lions, wolves, elephants, rabbits, guinea pigs, hamsters, rats, and mice.

The type of wound includes, but not limited to, acute surgical and traumatic wounds, bums (such as thermal, electrical bums, radiation, chemical, frost, and wind chill bums, as well as sunburns), diabetic ulcers, venous ulcers, arterial ulcers, pressure ulcers (otherwise known as decubitus ulcers or bedsores), skin ulcers of mixed etiologies such as ulcers caused by two or more of the disease selected from the group consisting of diabetes, cardiovascular disease, peripheral vascular disease, vasculitis, central or peripheral neuropathy, renal disease, autoimmune disease, and cancer, fistulas, skin fissures (caused by eczema, contact dermatitis, psoriasis, folliculitis, acne, lupus, herpes, etc.), and other chronic or necrotic wounds and inflammatory lesions and disorders. The materials according to the present invention are primarily intended for the treatment of both infected wounds and non-infected wounds (that is to say wounds showing no clinical signs of infection). The type of wound is not limited to an open wound of the skin, and may include intact skin but with inflammation or damage of the skin or the tissue underneath the skin, such as skin inflammation, fibrosis, dermatitis, erythema or edema due to irradiation (such as cancer radiotherapy). The dressing material can also be used prophylactically to treat skin damage by positioning the dressing on the target skin area to be affected, and then treating the target area with an energy therapy, so as to prevent tissue inflammation, fibrosis or other forms of injury caused by the energy therapy.

In one embodiment, a method is provided for treating a chronic wound of a human, which comprises: positioning the composite dressing material of the present invention in contact with the wound site and securing the dressing material to the skin of the human, wherein the human has had a non-healing wound for at least 2 months, optionally at least 3-6 months or at least 1 year. Optionally, this human subject has been previously treated with a wound care modality selected from the group consisting of living cell-based skin substitutes, skin grafts, acellular matrices, silver-containing dressing, an enzymatic debridement agent, growth factor, amniotic membrane, negative pressure wound therapy, ultrasound therapy, electrical stimulation therapy and hyperbaric oxygen therapy.

In another embodiment, a method is provided for treating a chronic wound of a human, which comprises: positioning the composite dressing material of the present invention in contact with the wound site and securing the dressing material to the skin of the human, wherein the size of the wound is reduced at least 50% in 4 weeks of the treatment.

The dressing material can be used as a primary dressing in direct contact with the wound bed throughout the whole treatment process. The user can conveniently apply the dressing material onto the wound bed, and secure it on the wound bed by using an adhesive bandage.

Alternatively, the dressing material of the prevent invention can be used in combination with other wound treatment modalities such as biological cell-based wound dressings, autologous or allogeneic skin grafts (including xenografts), acellular matrix, negative pressure wound therapy (NPWT), and hyperbaric oxygen therapy. The dressing material of the present invention can serve as a secondary dressing applied onto the primary dressing of skin grafts, biological cell-based wound dressings, acellular matrix, collagen-based dressings, etc. Such configuration is advantageous in that the inventive dressing material can provide a clean, moisture-balanced healing environment to protect the biological dressing from degradation or loss of viability due to microbial infection/contamination or due to desiccation of the wound bed.

Moreover, as the inventive dressing material is breathable, it can also be used under the foam dressing used in NPWT to protect the nascent regenerative wound bed from mechanical injury and from desiccation of the wound bed.

Also alternatively, the dressing material of the prevent invention can be used in combination with a growth factor for promoting tissue regeneration. The growth factor may be selected from the group consisting of epidermal growth factor (EGF), fibroblast growth factor (FGF), platelet derived growth factor (PDGF), transforming growth factor beta (TGF-beta), vascular endothelial growth factor (VEGF) and insulin-like growth factor (IGF), and mixtures thereof. The growth factor may be applied topically to the wound bed, or systemically to the patient with the wound. For example, the growth factor in a liquid form may be sprayed onto the wound bed directly, then followed by applying the inventive dressing material to the wound bed. Such a combination may be synergistic to further accelerate the wound healing and enhance the healing quality of the wound by further reducing scarring of deep wounds.

Also alternatively, the dressing material of the prevent invention can be used in combination with a silver-containing dressing. For example, the inventive dressing material can be applied to the wound directly, followed by application of a silver-containing dressing (such as a nano silver wound dressing, silver containing foam dressing, etc.) to further protect the wound from infection, and/or to absorb excessive exudates from the wound.

Also alternatively, the dressing material of the prevent invention can be broadly applied to plastic surgery and medical cosmetology. In many cases standard treatment of deep 2^{nd} degree or deeper bums, surgical or other types of wounds usually result in healing with scars. Patients often have to go through multiple rounds of reconstructive surgery post healing in order to minimize dysfunction of physiological motion, and to restore aesthetic appearance to the most extent. The dressing material of the prevent invention can be directly covered onto the scarred skin surface so as to promote renewing of the skin to facilitate sloughing off of the scar tissue. The dressing can also be used in combination with surgery, as well as other medical devices such as laser, infra-red radio frequency, sound wave, ultrasound, electromagnetic energy, mechanical dermabrasion, to synergistically achieve the best treatment results. In addition, the inventive dressing can also be used in combination with chemical peeling by applying the dressing onto the skin post chemical peeling (such as glycolic peeling) to quickly inhibit inflammation, provide an ideal local environment with physiological moisture balance, reduce thermal injury from the energy source and post-inflammatory hyperpigmentation, promote regenerative healing of the skin post scar revision and dermabrasion.

In yet another aspect of the present invention, a kit of skin or wound composite dressing is provided, comprising said composite dressing material and an outer packaging material enclosing the dressing material.

Further, the kit also comprises a protective sheet covering the wound-facing-side of the active layer of the composite dressing material, and/or an adhesive material for securing the dressing material to the skin of the mammal.

In one embodiment, the kit further comprises a first protective sheet covering the wound-facing side of the active layer of the composite dressing material. The protective sheet is removed prior to application of the composite dressing material to the wound. The protective sheet serves as an effective barrier between the wound-facing side of the active layer of the dressing and the outer packaging material.

Optionally the kit further comprises a second protective sheet covering the non-wound facing side of the active layer of the composite dressing material. The protective sheet serves as an effective barrier substantially non-permeable to gas, vapor or liquid, thereby extending the shelf life of the dressing material.

In another embodiment, the kit further comprises an exterior packaging housing enclosing the composite dressing material, such as a pouch for containing the dressing material. The pouch is removed prior to application of the dressing material to the wound. The exterior housing is preferred to be substantially non-permeable to gas, vapor or liquid, preferably a vacuum packaging pouch, thereby extending the shelf life of the dressing material. Examples of such an occlusive housing material includes, but are limited to general plastic sheets, polyester film, polyethylene terephthalate (PET or MYLAR) that can be clear, translucent, colored, or metalized with a thin layer of metal, usually aluminum, preferably a pouch made of multilayer composite aluminum-polyesterfoil.

For an exuding wound, the user may position an absorbent layer on the non-wound facing side of the active layer of the composite dressing material to absorb excessive wound fluids, serum or blood from the wound, including gauzes, nonwoven fabrics, superabsorbents, hydrogels, absorbent foams, such as polyurethane foam.

In a further aspect, the present invention provides a method of manufacture of the composite dressing material of the present invention, comprising the steps of: impregnating a porous, polymeric substrate with a semi-solid hydrophobic composition comprising fatty acid and solid inorganic particles such that the solid inorganic particles are substantially evenly embedded in the matrix of the polymeric substrate.

According to this method, the step of impregnating the porous, polymeric substrate may be conducted at a temperature above 50°C, optionally above 60°C or 80°C, at which the semi-solid hydrophobic composition is substantially liquefied. The step of impregnating the porous, polymeric substrate may be achieved by a precisely controlled mechanical manufacturing process that substantially evenly spreads the liquefied hydrophobic composition onto a sheet of the porous, polymeric substrate such that the average thickness of the hydrophobic composition impregnated in the substrate is about 0.1-10 mm, optionally 0.2-5 mm, or 1-4 mm.

It will be appreciated that any feature or embodiment that is described herein in relation to any one aspect of the invention may also be applied to any other aspect of the invention.

Certain specific embodiments of the present invention will now be described further in the following examples.

### Examples

### Example 1

Figure 3 shows treatment of a patient with burn wounds using the dressing material of the present invention: male, age 10; sustained deep 2^{nd} degree scalds by boiling water on the left ankle; wound condition worsened in a prior treatment for 4 days using a burn ointment; then treated with the inventive dressing; dressing change every 1-2 days. Figure 3A shows that prior to the inventive treatment there was a large amount of necrotic tissue and eschar with erythema and inflammation of the wounds and the periwound areas. After one day of treatment using an embodiment of the inventive dressing, the eschar on the wounds substantially liquefied through autolytic debridement (Figure 3B). Upon cleansing of the wounds using saline, the wounds were clean and left with a thin layer of crust (Figure 3C). Inflammation of the wounds and the periwound areas significantly subsided. After 14 days of treatment, the wounds healed completely without obvious scarring (Figures 3D, E, F, G).

### Example 2

Figure 4 shows treatment of a patient with burn wounds using the dressing material of the present invention: male, age 1 year and 5 months; sustained deep 2^{nd} and 3^{rd} degree scalds by boiling water on the right arm and hand; wound condition worsened in a prior treatment for 16 days using a burn ointment (and also due to exposure of wounds in the air in winter time resulting in tissue necrosis); high fever for several days; then treated with the inventive dressing; dressing change every 1-2 days. Figure 4A shows that prior to the inventive treatment there was a large amount of necrotic tissue and hard eschar with erythema and inflammation of the wounds and the periwound areas, coupled with systemic inflammatory reactions. After one day of local treatment using an embodiment of the inventive dressing, coupled with systemic treatment with antibiotics and anti-inflammatory drugs, the hard eschar on the wounds substantially liquefied through autolytic debridement; upon cleansing of the wounds using saline, the wounds were clean (Figure 4B) with fresh granulation tissue appearing after 4 days of treatment (Figure 4C). Inflammation of the wounds and the periwound areas significantly subsided. After 46 days of treatment, the wounds healed completely without skin grafting (Figures 4D, E, F).

### Example 3

Figures 5 and 6 show treatment of a mother and her son, both simultaneously sustained deep scalds by boiling water, by using the dressing material of the present invention: The mother, age 32; sustained deep scalds on the right foot and ankle; wound condition worsened to become 3^{rd} degree wounds in a prior treatment for 18 days using two kinds of burn ointment; then treated with the inventive dressing; dressing change every 1-2 days. Figure 5A that shows prior to the inventive treatment there was a large amount of necrotic tissue, eschars and ulcers beneath thereof, with erythema and inflammation of the wound and the periwound area. After one day of local treatment using an embodiment of the inventive dressing, the eschars on the wounds substantially softened and liquefied; upon cleansing of the wounds using saline and removal of the softened eschars, the wounds were clean (Figure 5B) with fresh granulation tissue appearing; and inflammation of the wounds and the periwound areas significantly subsided. After 17 days of treatment, the wounds healed completely without obvious scarring (Figures 5C, D, E, F). The son, age 3; sustained deep scalds on the right foot and ankle to a similar degree of his mother and went through the same prior treatment as his mother; wound condition worsened to become 3^{rd} degree wounds using two kinds of burn ointment (Figure 6A); then treated with the inventive dressing following a similar process. After one day of local treatment using an embodiment of the inventive dressing, the eschars on the wounds substantially liquefied via autolytic debridement; upon cleansing of the wounds using saline, the wounds were clean (Figure 6B). After 23 days of treatment, the wounds healed completely without obvious scarring (Figures 6C, D, E, F).

### Example 4

Figure 7 shows treatment of a patient with burn wounds using an embodiment of the invention: male, age 4; sustained deep 2^{nd} degree scalds by boiling water on the left lower leg and foot; wound condition worsened in a prior treatment for 15 days using another burn therapy; then treated with the inventive dressing; dressing change every 1-2 days. Figure 7A shows that prior to the inventive treatment there was a large amount of necrotic tissue and black eschars with erythema and inflammation of the wounds and the periwound areas. After one day of treatment using an embodiment of the inventive dressing, some eschars on the wounds liquefied through autolytic debridement while the rest of the unliquefied black eschars were removed upon change of the dressings (Figure 7C). The wounds were left very clean (Figure 7B); and inflammation of the wounds and the periwound area significantly subsided. After 6 days of treatment, 70% of the wounds healed (Figures 7D, E, F).

### Example 5

Figure 10 shows treatment of a patient with chronic wounds using an embodiment of the invention: male, age 61; with a 10-year history of varicose veins; skin infected due to scratching 2 years ago, gradually developing enlarging and deepening non-healing chronic ulcers; ulcer condition worsened due to a recent collision rendering further enlargement of the wound area, resulting in painful claudication; ulcers non-healing by prior treatment; then treated with the inventive dressing; dressing change every 2-3 days. Figure 10A shows that prior to the inventive treatment there was a large amount of necrotic tissue, sloughs, fibrous eschar with erythema and hyperpigmentation of the periwound areas. After 2 days of treatment using an embodiment of the inventive dressing, eschars on the wounds liquefied through autolytic debridement and were removed upon change of the dressings (Figure 10C). The wounds were left very clean (Figure 10B); and inflammation of the wounds and the periwound area significantly subsided. Figure 10C shows that the liquefied necrotic tissue was compartmentally locked in the dressing area, and was prevented to diffuse into the surrounding areas with shallower, less-exuding wounds. Figure 10D shows that after 12 days of treatment, granulation tissue on wound bed appear to be fresh and alive, and the exudates infiltrated vertically and were absorbed by the dressing (Figure 10E). Figure 10F shows that on the dressing removed from the wound the exudates were compartmentally locked in the dressing, preventing them from diffusing into the surrounding peri-wound area. After 32 days of treatment, the wounds substantially healed (Figures 10G, H).

### Example 6

Figure 11 shows treatment of a patient with burn wounds using an embodiment of the inventive dressing material: female, age 3; sustained deep 2^{nd} degree scalds by boiling water on the rear waist, buttocks, and both lower limbs; wound condition worsened in a prior treatment for 1 day using a burn ointment; then treated with the inventive dressing; dressing change every 1-2 days. Figure 11A shows that prior to the inventive treatment there was a large amount of blister skin and necrotic tissue with erythema and inflammation of the wounds and the periwound areas. After 15 days of treatment using the inventive dressing, the blister skin and necrotic tissue on the wounds were debrided via autolytic debridement, and the wounds essentially healed (Figures 11B, C) with regeneration of dermis and epidermis without skin grafting. After 23 days of treatment, the wounds healed completely (Figure 11D). In a follow-up visit 38 days later it was observed that the healed skin resembled that healed via physiological regeneration without obvious scarring.

### Example 7

Figure 13 shows treatment of a patient with a mechanically injured wound using an embodiment of the inventive dressing material: female, age 6; her right heel sustained a muscle-reaching laceration by spokes of the wheel of a motorcycle; wound condition worsened after treatment with a wound ointment for 20 days; then treated with the inventive dressing; dressing change every 1-2 days. Figure 13A shows that prior to the inventive treatment there was a large amount of hard, fibrotic necrotic tissue with erythema and inflammation of the wounds and the periwound areas. The inventive dressing was applied to the wound (Figure 13B), and secured with a regular gauze bandage (Figure 13C). After 2 days of treatment using the inventive dressing, the hard eschars on the wound softened and liquefied through autolytic debridement and were removed upon dressing change, leaving a very clean wound (Figure 13D) with erythema and inflammation of the wound and the periwound area subsided. After 40 days of treatment, dermis and epidermis were regenerated; and the wounds essentially healed without skin grafting (Figures 13E, F). Figure 13F shows that the healing is close to physiological healing without obvious scarring.

### Example 8

An embodiment of the composite dressing material of the present invention can be manufactured by the following protocol: adding solid particles of zinc oxide and calcium carbonate that have been both ground to a diameter below 200 µm at 1:1 weight ratio to vegetable oil such that each of the inorganic compounds in this mixture is about 20-30% by weight; Healing and mixing this mixture with stirring at 80-120°C, then adding melt beeswax at 2-8% by weight based on the total weight of the final mixture; filtering the final mixture through a screen filter of 120-240 mesh, and allowing the filtrate to cool down to room temperature to produce a semi-solid hydrophobic composition that is subsequently heated to liquefy at 70-120°C and impregnated by a machine into a layer or multiple layers of nonwoven rayon/polyester substrate; cooling the impregnated substrate to room temperature to produce a thin-layer type of composite dressing material that can be cut into various shapes and sizes, and subsequently seal packaged.

### Example 9

Another embodiment of the composite dressing material of the present invention can be manufactured by the following protocol: adding solid particles of zinc oxide and calcium sulfate that have been both ground to a diameter below 80 µm at 1:1 weight ratio to a mixture of olive oil and sesame oil such that each of the inorganic compounds in this mixture is about 20-30% by weight; Healing and mixing this mixture with stirring at 90-110°C, then adding melt beeswax at 2-8% by weight based on the total weight of the final mixture; filtering the final mixture through a screen filter of 200 meshes, and allowing the filtrate to cool down to room temperature to produce a semi-solid hydrophobic composition that is subsequently heated to liquefy at 80-100°C and impregnated by a machine into a layer or multiple layers of nonwoven rayon/polyester substrate; cooling the impregnated substrate to room temperature to produce a thin-layer type of composite dressing material.

### Example 10

Yet another embodiment of the composite dressing material of the present invention can be manufactured by the following protocol: adding solid particles of zinc carbonate and calcium sulfate that have been both ground to a diameter below 100 µm at 1:1 weight ratio to a mixture of rapeseed oil and olive oil such that each of the inorganic compounds in this mixture is about 20-30% by weight; Healing and mixing this mixture with stirring at 80-120°C, then adding melt beeswax at 2-8% by weight based on the total weight of the final mixture; filtering the final mixture through a screen filter of 120-240 meshes, and allowing the filtrate to cool down to room temperature to produce a semi-solid hydrophobic composition that is subsequently heated to liquefy at 80-120°C and impregnated by a machine into a layer or multiple layers of nonwoven rayon/bamboo viscose/polyester substrate; cooling the impregnated substrate to room temperature to produce a thin-layer type of composite dressing material that can be cut into various shapes and sizes, and subsequently vacuum seal packaged.

### Example 11

Yet another embodiment of the composite dressing material of the present invention can be manufactured by the following protocol: adding solid particles of zinc carbonate and calcium sulfate that have been both ground to a diameter below 100 µm at 1:1 weight ratio to camellia seed oil such that each of the inorganic compounds in this mixture is about 20-30% by weight; Healing and mixing this mixture with stirring at 80-120°C, then adding melt beeswax at 2-8% by weight based on the total weight of the final mixture; filtering the final mixture through a screen filter of 120-240 meshes, and allowing the filtrate to cool down to room temperature to produce a semi-solid hydrophobic composition that is subsequently heated to liquefy at 70-120°C and impregnated by a machine into a layer or multiple layers of nonwoven bamboo viscose substrate; cooling the impregnated substrate to room temperature to produce a thin-layer type of composite dressing material that can be cut into various shapes and sizes, and subsequently seal packaged.

### Example 12

Yet another embodiment of the composite dressing material of the present invention can be manufactured by the following protocol: adding solid particles of zinc carbonate and calcium sulfate that have been both ground to a diameter below 100 µm at 1:1 weight ratio to a mixture of rapeseed oil and camellia seed oil such that each of the inorganic compounds in this mixture is about 20-30% by weight; Healing and mixing this mixture with stirring at 70-120°C, then adding melt beeswax at 3-10% by weight based on the total weight of the final mixture; filtering the final mixture through a screen filter of 120-240 meshes, and allowing the filtrate to cool down to room temperature to produce a semi-solid hydrophobic composition that is subsequently heated to liquefy at 70-120°C and impregnated by a machine into a layer or multiple layers of nonwoven bamboo viscose substrate; cooling the impregnated substrate to room temperature to produce a thin-layer type of composite dressing material that can be cut into various shapes and sizes, and subsequently vacuum seal packaged.

### Example 13

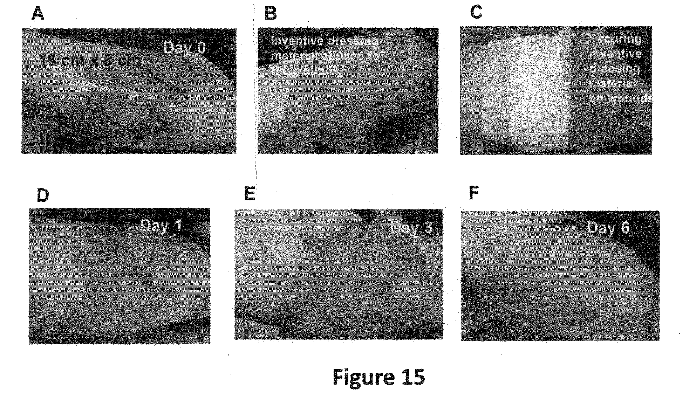

Figure 15 shows treatment of a patient with burn wounds with an embodiment of the inventive dressing material: Male, age 32; 2^{nd} degree scald bums of left thigh by boiling water; previously treated w/ a burn ointment for 5 days without improvement; and subsequently treated with an embodiment of the inventive dressing material with dressing changed every 1-2 days; After 6 days of treatment the wounds healed completely without obvious scarring.

### Example 14

Figure 16 shows treatment of a patient with burn wounds with an embodiment of the inventive dressing material: Male, age 1 year and 6 months; 2^{nd} Degree scald bums of right foot by boiling water; and subsequently treated with an embodiment of the inventive dressing material with dressing changed every 1-2 days. After 7 days of treatment the wounds healed completely without obvious scarring.

### Example 15

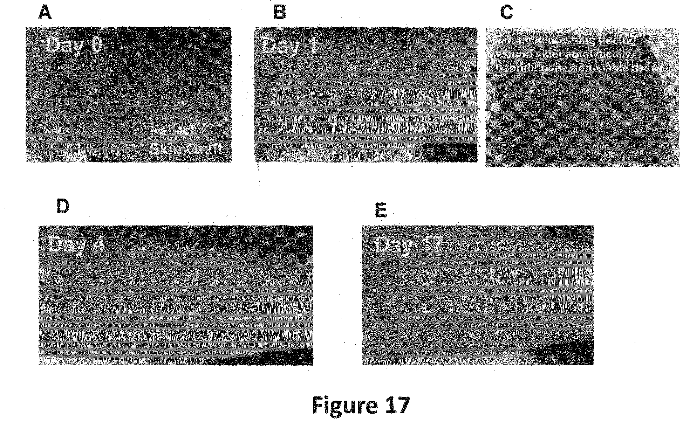

Figure 17 shows treatment of a patient with burn wounds with an embodiment of the inventive dressing material: Female, age 5 years 6 months; 2^{nd} - 3^{rd} Degree bums of left inner thigh and right hand by boiling water 14 days ago; treated in another hospital with other modalities and skin grafting failed; and subsequently treated with an embodiment of the inventive dressing material with dressing changed every 1-2 days. After 17 days of treatment the wounds essentially healed without obvious scarring.

### Example 16

Figure 18 shows treatment of a patient with chemical bums using an embodiment of the inventive dressing material: male, age 58; right lower leg fell into a tank containing a high temperature (130°C/266°F) alkaline liquid, rescued a few minutes later and subsequently hospitalized in 3 hours; sustained a deep 2^{nd} and 3^{rd} degree bums (TBSA of 13%); standard systemic burn management employed; then topically treated with the inventive dressing; dressing change every 1-2 days. After 19 days of treatment the wounds essentially healed without obvious scarring.

### Example 17

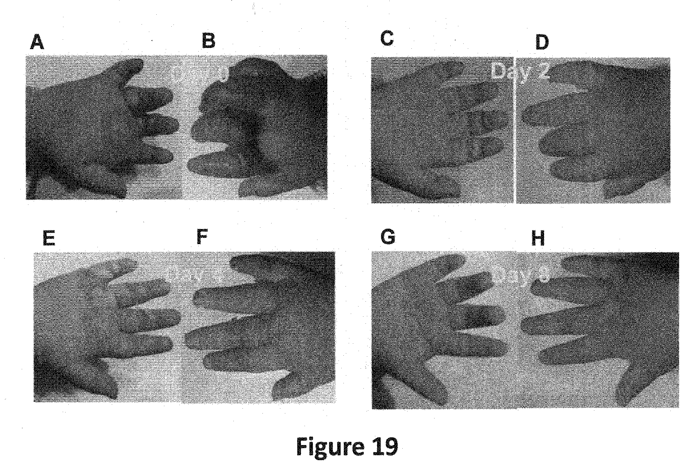

Figure 19 shows treatment of a patient with charcoal burn wounds with an embodiment of the inventive dressing material: Male, age 4; deep 2^{nd} degree bums of right hand by burning charcoal; treated with a burn ointment for 3 days but condition worsened; and subsequently treated with an embodiment of the inventive dressing material with dressing changed every 1-2 days. After 8 days of treatment the wounds essentially healed without obvious scarring.

### Example 18

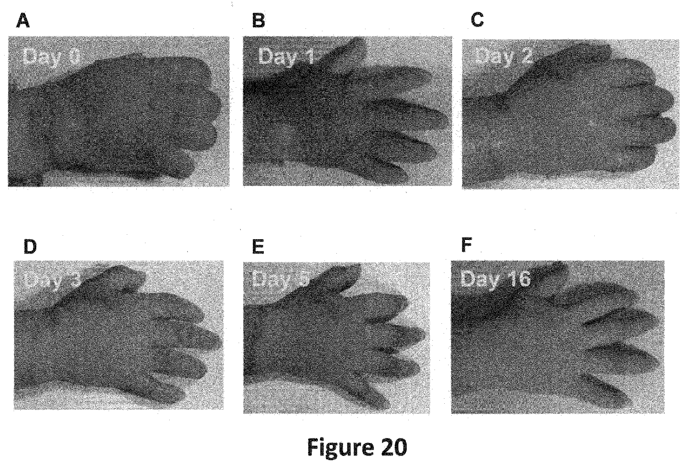

Figure 20 shows treatment of a patient with burn wounds with an embodiment of the inventive dressing material: Male, age 3; deep 2^{nd} degree bums of right hand by boiling water; treated with a burn ointment for 1 day but condition worsened; and subsequently treated w/ an embodiment of the inventive dressing material with dressing changed every 1-2 days. After 5 days of treatment the wounds essentially healed without obvious scarring.

### Example 19

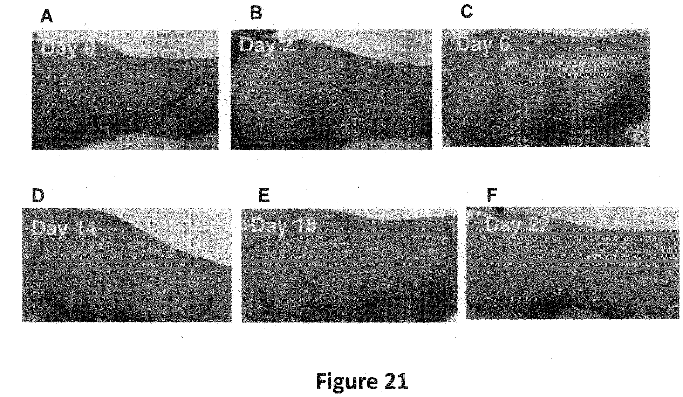

Figure 21 shows treatment of a patient with burn wounds with an embodiment of the inventive dressing material: Female, age 1 yr 1 mon; deep 2^{nd} degree bums of right leg by boiling water; delayed undressing worsened the condition; and subsequently treated with an embodiment of the inventive dressing material with dressing changed every 1-2 days. After 22 days of treatment the wounds essentially healed without obvious scarring.

### Example 20

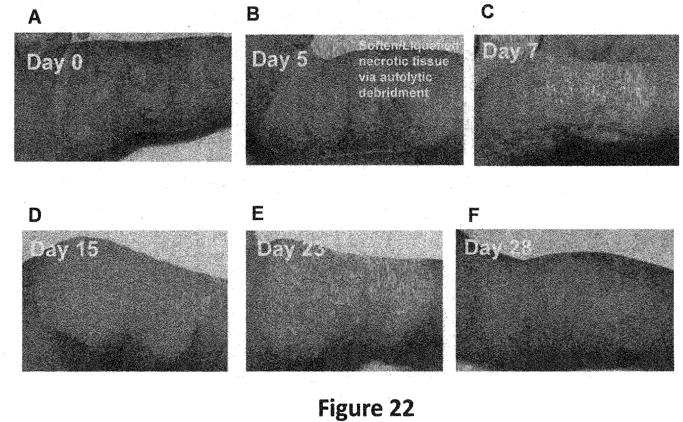

Figure 22 shows treatment of a patient with burn wounds with an embodiment of the inventive dressing material: Female, age 1 year 2 months; 2^{nd} - 3^{rd} degree bums of left knee and below by boiling water 1 day ago; and subsequently treated with an embodiment of the inventive dressing material with dressing changed every 1-2 days. After 28 days of treatment the wounds essentially healed without obvious scarring.

### Example 21

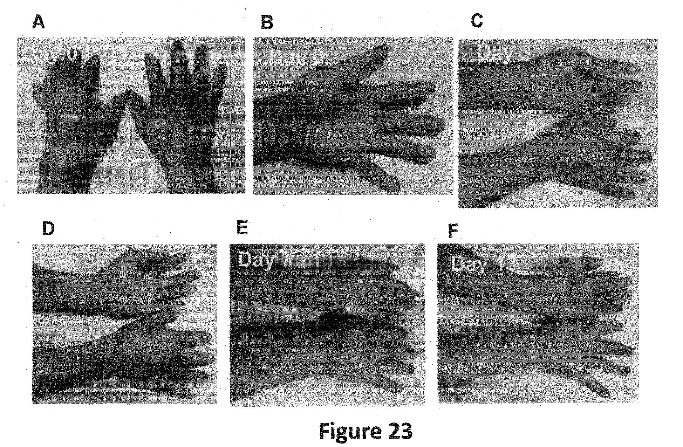

Figure 23 shows treatment of a patient with electrical burn wounds with an embodiment of the inventive dressing material: Male, age 39, electrician; electrical bums of both hands due to circuit shorting; and subsequently treated with an embodiment of the inventive dressing material with dressing changed daily. After 13 days of treatment the wounds essentially healed without obvious scarring.

### Example 22

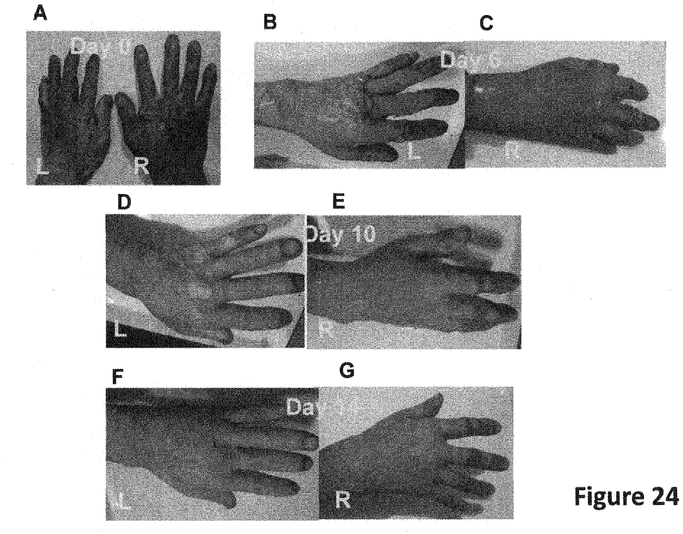

Figure 24 shows treatment of a patient with electrical burn wounds with an embodiment of the inventive dressing material: Male, age 60, electrician; high voltage electrical bums on both hands (2^{nd} - 3^{rd} Degree) and the face (2^{nd} Degree); and subsequently treated with an embodiment of the inventive dressing material with dressing changed daily. After 14 days of treatment the wounds essentially healed without obvious scarring.

### Example 23

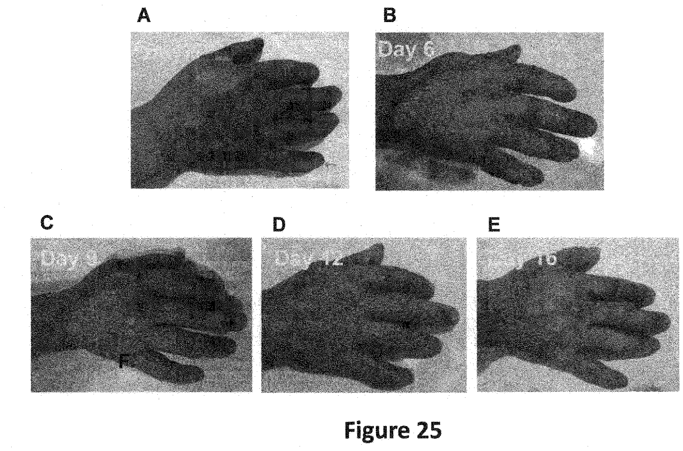

Figure 25 shows treatment of a patient with electrical burn wounds with an embodiment of the inventive dressing material: Male, age 52; electrician, high voltage electrical bums on right hand; and subsequently treated with an embodiment of the inventive dressing material with dressing changed daily. After 16 days of treatment the wounds essentially healed without obvious scarring.

### Example 24

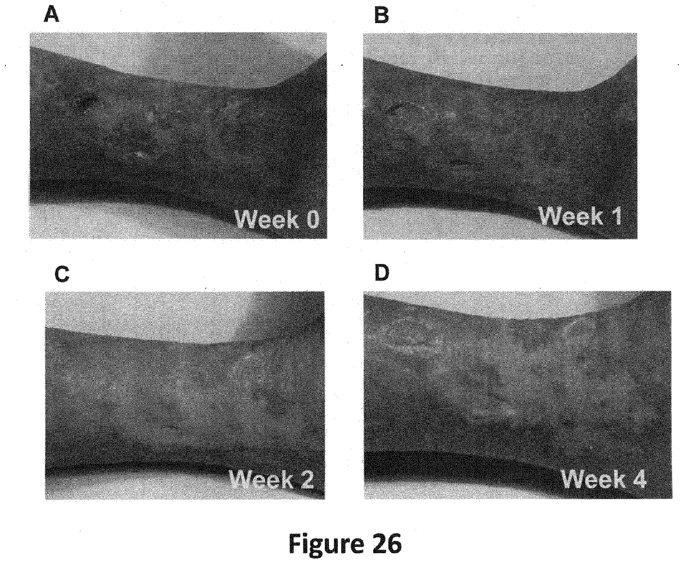

Figure 26 shows treatment of a patient with venous leg ulcers with an embodiment of the inventive dressing material: Female, age 75; having venous stasis of left lower extremity for 29 years; 2 years ago insect bites led to non-healing ulcers; and subsequently treated with an embodiment of the inventive dressing material with dressing changed every 2-3 days. After 4 weeks of treatment about 80% of the wounds healed.

### Example 25

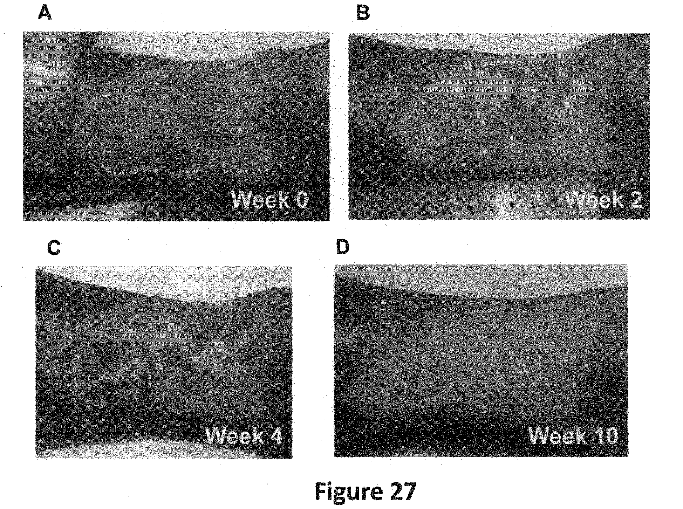

Figure 27 shows treatment of a patient with venous leg ulcers with an embodiment of the inventive dressing material: Male, age 67; having venous stasis of left lower extremity for more than 20 years; 2 years ago sustained an impact injury that led to a non-healing ulcer that failed treatment of other therapies; and subsequently treated with an embodiment of the inventive dressing material with dressing changed every 1-2 days. After 10 weeks of treatment the wounds healed completely.

### Example 26

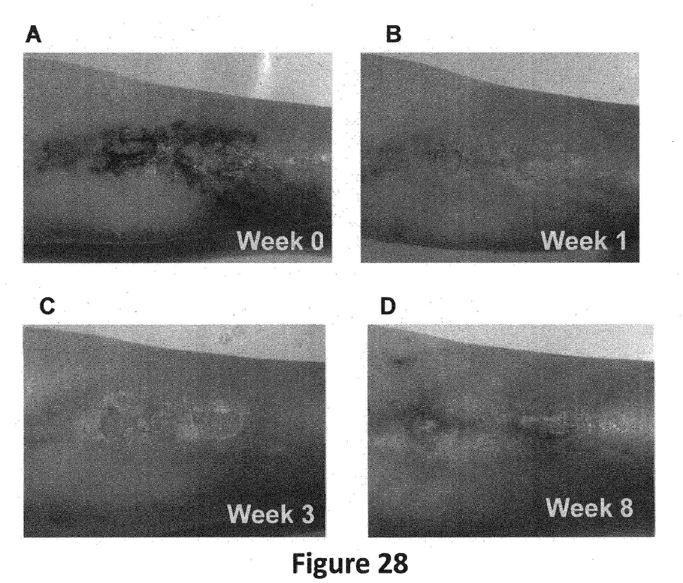

Figure 28 shows treatment of a patient with a non-healing surgical wound with an embodiment of the inventive dressing material: Male, age 39; twenty years ago sustained an auto accident causing open tibia fractures of the right lower leg; removal of the steel implant resulted in non-healing wounds since then; and subsequently treated with an embodiment of the inventive dressing material with dressing changed every 2-3 days. After 8 weeks of treatment the wound essentially healed.

### Example 27

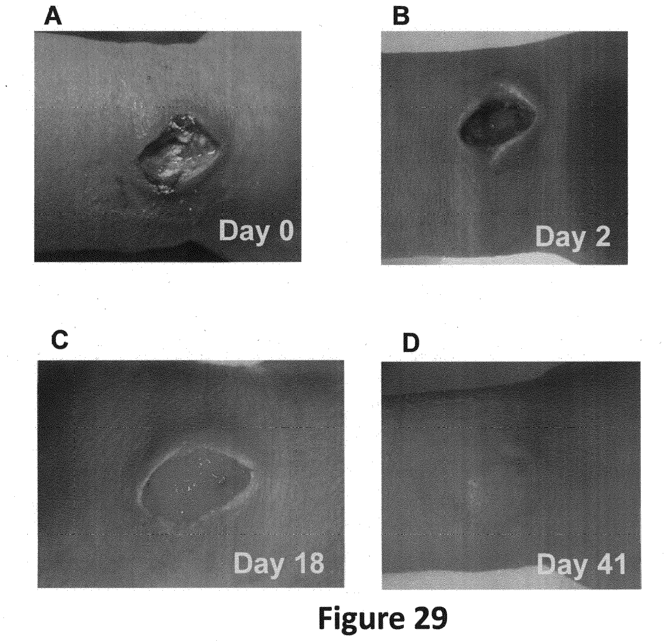

Figure 29 shows treatment of a patient with a non-healing surgical wound with an embodiment of the inventive dressing material: Female, age 38; having surgical wound on right wrist that failed to heal 18 days post ganglion cyst surgery; became infected and necrotic; and subsequently treated with an embodiment of the inventive dressing material with dressing changed every 1-2 days. After 6 weeks of treatment the wound essentially healed.

### Example 28

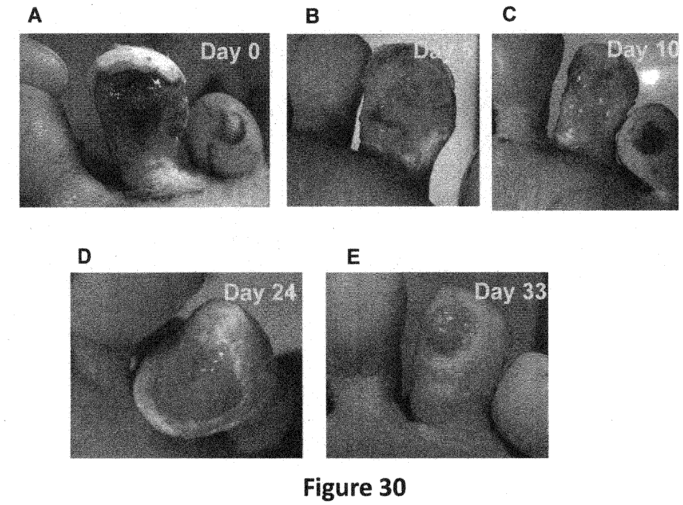

Figure 30 shows treatment of a patient with a non-healing surgical wound with an embodiment of the inventive dressing material: Male, age 28; excision of skin cancer on 4^{th} toe of left foot left a non-healing wound developing necrosis in the course of 20 days; Amputation of the toe was offered at another hospital but he declined; and subsequently treated with an embodiment of the inventive dressing material with dressing changed every 1-2 days. After 5 weeks of treatment 70% of the wounds healed.

### Example 29

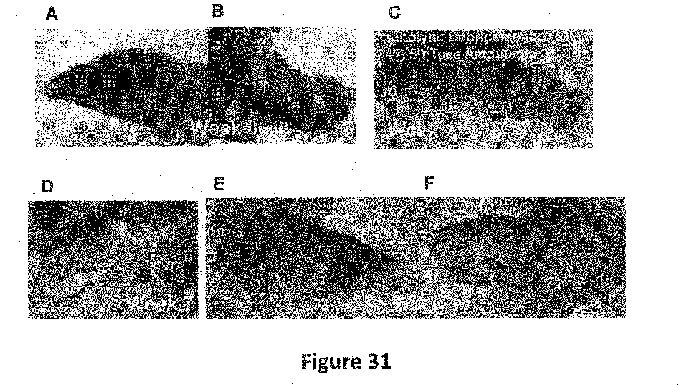

Figure 31 shows treatment of a patient with a foot gangrene with an embodiment of the inventive dressing material: Female, age 70; having coronary atherosclerosis, coronary arterial disease, atrial fibrillation, hyperlipidemia for more than 10 years; having severe pain of right toes developed 6 months ago; dry gangrene developed in right foot; and subsequently treated with an embodiment of the inventive dressing material with dressing changed every 1-2 days. After 15 weeks of treatment 70% of the wounds healed.

While the foregoing description is exemplary of the preferred embodiment of the present invention, those of ordinary skill in the relevant arts will recognize the many variations, alterations, modifications, substitutions and the like are readily possible, especially in light of this description and the accompanying drawings. In any case, because the scope of the present invention is much broader than any particular embodiment, the foregoing detailed description should not be construed as a limitation of the scope of the present invention, which is limited only by the claims that are drawn hereto.

## Claims

1. A multifunctional skin or wound composite dressing as a regenerative skin substitute, comprising: an active layer of a porous, polymeric substrate impregnated with a semi-solid hydrophobic composition, wherein the semi-solid hydrophobic composition comprises fatty acid and solid inorganic particles embedded in the matrix of the polymeric substrate.

2. The composite dressing according to claim 1, wherein the semi-solid hydrophobic composition is mechanically processed so as to allow the fatty acid to substantially evenly soak through the matrix of the polymeric substrate, and the solid inorganic particles substantially evenly get embedded in the matrix of the polymeric substrate.

3. The composite dressing according to claim 2, wherein the semi-solid hydrophobic composition is substantially evenly impregnated in the matrix of the polymeric substrate so as to form a layer of 0.1-10 mm in thickness.

4. The composite dressing according to claim 1, wherein the polymeric substrate is a material with a net structure comprising fiber bundles, each of the fiber bundles composed of multiple microfilaments.

5. The composite dressing according to claim 4, wherein there is interstitial space among the multiple microfilaments.

6. The composite dressing according to claim 4, wherein the longest cross diameter of the microfilament is 0.01-20 µm.

7. The composite dressing according to claim 1, wherein the porous polymeric substrate is woven or non-woven fiber selected from the group consisting of cotton, silk, linen, polyester, nylon, polyamide, polypropylene, polyurethane, polytetrafluoroethylene, rayon, bamboo fiber, bamboo viscose, corn, soy, alginate, chitin, chitosan, hyaluronan, and animal protein.

8. The composite dressing according to claim 1, wherein the semi-solid hydrophobic composition comprises less than 5% of water in weight based on the total weight of the dressing material.

9. The composite dressing according to claim 1, wherein the semi-solid hydrophobic composition further comprises one or more of a solidifier, a bioabsorbable composition or hydrogel.

10. The composite dressing according to claim 1, wherein the concentration of the fatty acid is 20-80% by weight based on the total weight of the dressing material.

11. The composite dressing according to claim 1, wherein the solid inorganic particles are one or more of the inorganic compounds selected from the group consisting of talc, silicon dioxide, aluminum oxide, magnesium oxide, zinc oxide, iron oxides, calcium carbonate, calcium chloride, calcium, calcium sulfate, and titanium dioxide.

12. The composite dressing according to claim 1, wherein the average size of the particles is 0.01 - 50 µm, or at least 80% of the solid inorganic particles has the size of less than 50 or 30 µm.

13. The composite dressing according to claim 1, wherein the concentration of the solid particles in the dressing material is 20-80% by weight based on the total weight of the dressing material.

14. The composite dressing according to claim 1, wherein the composite dressing material is capable of accelerating softening or liquefying necrotic tissue or eschar on the wound, thereby resulting in autolytic debridement of the wound within 1-4 days.

15. A kit of skin or wound composite dressing, comprising: said composite dressing material according to any of claims 1-14, and an outer packaging material enclosing the dressing material.

16. The kit according to claim 15, further comprising a protective sheet covering the wound-facing-side of the active layer of the composite dressing, and/or an adhesive material for securing the dressing to the skin of a mammal.

17. A method of manufacturing a skin or wound composite dressing, **characterized by** the feature of the method comprising the step of comprising the step of: impregnating a porous, polymeric substrate with a semi-solid hydrophobic composition comprising fatty acid and solid inorganic particles such that the solid inorganic particles are substantially evenly embedded in the matrix of the polymeric substrate.

18. The method according to claim 17, wherein the method comprises the steps of grinding the solid inorganic particles; and adding the ground particles to the fatty acid; heating and mixing well so as to produce a hydrophobic composition that is semi-solid at room temperature; heating and liquefying the semi-solid hydrophobic composition; and substantially evenly spreading the liquefied hydrophobic composition onto one or more sheets of the porous, polymeric substrate, producing the composite dressing upon cooling to room temperature.

19. A method for treating the skin or a wound of a mammal using a composite dressing, comprising: positioning the composite dressing of claim 1 in contact with the wound site; and securing the dressing to the skin of the mammal.

20. The method according to claim 19, wherein the wound is one or more types of wounds selected from the group consisting of acute surgical and traumatic wounds, bums, scalds, thermal skin injuries, chemical bums, electrical bums, sunburns, frost, diabetic ulcers, venous ulcers, arterial ulcers, pressure ulcers, skin ulcers of mixed etiologies, fistulas, skin inflammation, herpes, and fissures.
